# EUROPEAN PATENT SPECIFICATION

(11) **EP 1 904 108 B1**
(45) Date of publication and mention of the grant of the patent: **30.11.2011**
(21) Application number: 06787575.7
(22) Date of filing: 14.07.2006
(51) Int. Cl.: A61K 45/06, A61K 31/10, A61K 47/16, A61K 31/195, A61K 38/05, A61K 31/573, A61K 31/495, A61P 27/02, A61P 27/06, A61P 27/12

(54) **OPHTHALMOLOGICAL FORMULATION COMPRISING METHYLSULFONYLMETHANE AND CIPROFLOXACIN**
OPHTHALMOLOGISCHE FORMULIERUNG MIT METHYLSULFONYLMETHAN UND CIPROFLOXACIN
FORMULATION OPHTHALMOLOGIQUE COMPRENANT DU METHYLSULFONYLMETHANE AND CIPROFLOXACINE

(30) Priority: 15.07.2005 US 182998; 15.07.2005 US 183479
(43) Date of publication of application: 02.04.2008
(73) Proprietor: Chakshu Research, Inc., Los Gatos, CA 95032 (US)
(72) Inventor: BHUSHAN, Rajiv, Palo Alto, California 94306 (US); GIN, Jerry, B., Sunnyvale, California 94087 (US)
(74) Representative: Mallalieu, Catherine Louise
(86) International application number: PCT/US2006/027685
(87) International publication number: WO 2007/011874

(56) References cited:
- WO-A-2004/058289
- WO-A2-2004/087043
- US-A- 5 817 630
- US-A1- 2004 137 068
- UMIT UBEYT INAN ET AL: "Prevention of posterior capsule opacification by intraoperative single-dose pharmacologic agents" JOURNAL CATARACT AND REFRACTIVE SURGERY, AMERICAN SOCIETY OF CATARACT AND REFRACTIVE, US, vol. 27, no. 7, 2001, pages 1079-1087, XP002979447 ISSN: 0886-3350
- NAGAI K ET AL: "Die hemmende Wirkung von Carnosin auf verschiedene allergische Reaktionen", ARZNEIMITTEL-FORSCHUNG AUG 1971 LNKD- PUBMED:4255534, vol. 21, no. 8, August 1971 (1971-08), pages 1222-1225, XP9142619, ISSN: 0004-4172
- NAGAI K ET AL: 'Die hemmende Wirkung von Carnosin auf verschiedene allergische Reaktionen' ARZNEIMITTEL-FORSCHUNG AUG 1971 LNKD- PUBMED:4255534 vol. 21, no. 8, August 1971, pages 1222 - 1225, XP009142619 ISSN: 0004-4172

## Description

### TECHNICAL FIELD

This invention relates generally to the use of a permeation enhancer for treatment of disorders, diseases, and other adverse medical conditions, including the adverse ocular conditions disorders often associated with aging.

### BACKGROUND ART

Progressive, age-related changes of the eye, including normal as well as pathological changes, have always been an unwelcome but inevitable part of extended life in humans and other mammals. Many of these changes seriously affect both the function and the cosmetic appearance of the eyes. These changes include: development of cataracts; hardening, opacification, reduction of pliability, and yellowing of the lens; yellowing and opacification of the cornea; presbyopia; clogging of the trabeculum, leading to intraocular pressure build-up and glaucoma; increased floaters in the vitreous humor; stiffening and reduction of the dilation range of the iris; age-related macular degeneration (AMD); formation of atherosclerotic deposits in retinal arteries; dry eye syndrome; and decreased sensitivity and light level adaptation ability of the rods and cones of the retina. Age-related vision deterioration includes loss in visual acuity, visual contrast, color and depth perception, lens accommodation, light sensitivity, and dark adaptation. Age-related changes also include changes in the color appearance of the iris, and formation of arcus senilis. The invention is, in large part, directed toward a formulation and method for preventing and treating a multiplicity of age-related ocular disorders and diseases.

All parts of the eye, including the cornea, sclera, trabeculum, iris, lens, vitreous humor, and retina are affected by the aging process, as explained below.

### The Cornea:

The cornea is the eye's outermost layer. It is the clear, dome-shaped surface that covers the front of the eye. The cornea is composed of five layers. The epithelium is a layer of cells that forms the surface. It is only about 5-6 cell layers thick and quickly regenerates when the cornea is injured. If an injury penetrates more deeply into the cornea, scarring may occur and leave opaque areas, causing the cornea to lose its clarity and luster. Immediately below the epithelium is Bowman's membrane, a protective layer that is very tough and difficult to penetrate. The stroma, the thickest layer of the cornea, lies just beneath Bowman's membrane and is composed of tiny collagen fibrils aligned in parallel, an arrangement that provides the cornea with its clarity. Descemet's membrane underlies the stroma and is just above the innermost corneal layer, the endothelium. The endothelium is just one cell layer in thickness, and serves to pump water from the cornea to the aqueous, keeping it clear. If damaged or diseased, these cells will not regenerate.

As the eye ages, the cornea can become more opaque. Opacification can take many forms. The most common form of opacification affects the periphery of the cornea, and is termed "arcus senilis," or "arcus." This type of opacification initially involves deposition of lipids into Descemet's membrane. Subsequently, lipids deposit into Bowman's membrane and possibly into the stroma as well. Arcus senilis is usually not visually significant, but is a cosmetically noticeable sign of aging. There are other age related corneal opacifications, however, which may have some visual consequences. These include central cloudy dystrophy of Francois, which affects the middle layers of the stroma, and posterior crocodile shagreen, which is central opacification of the posterior stroma. Opacification, by scattering light, results in progressive reduction of visual contrast and visual acuity.

Opacification of the cornea develops as a result of a number of factors, including, by way of example: degeneration of corneal structure; cross-linking of collagen and other proteins by metalloproteinases; ultraviolet (UV) light damage; oxidation damage; and buildup of substances like calcium salts, protein waste, and excess lipids.

There is no established treatment for slowing or reversing corneal changes other than surgical intervention. For example, opaque structures can be scraped away with a blunt instrument after first removing the epithelium, followed by smoothing and sculpting the corneal surface with a laser beam. In severe cases of corneal scarring and opacification, corneal transplantation has been the only effective approach.

Another common ocular disorder that adversely affects the cornea as well as other structures within the eye is keratoconjunctivitis sicca, commonly referred to as "dry eye syndrome" or "dry eye." Dry eye can result from a host of causes, and is frequently a problem for older people. The disorder is associated with a scratchy sensation, excessive secretion of mucus, a burning sensation, increased sensitivity to light, and pain. Dry eye is currently treated with "artificial tears," a commercially available product containing a lubricant such as low molecular weight polyethylene glycol. Surgical treatment, also, is not uncommon, and usually involves insertion of a punctal plug so that lacrimal secretions are retained in the eye. However, both types of treatment are problematic: surgical treatment is invasive and potentially risky, while artifical tear products provide only very temporary and often inadequate relief.

### The sclera:

The sclera is the white of the eye. In younger individuals, the sclera has a bluish tinge, but as people grow older, the sclera yellows as a result of age-related changes in the conjunctiva. Over time, UV and dust exposure may result in changes in the conjunctival tissue, leading to pingecula and pterygium formation. These ocular growths can further cause breakdown of scleral and corneal tissue. Currently, surgery, including conjunctival transplantation, is the only accepted treatment for pingeculae and pterygia.

### The trabeculum:

The trabeculum, also referred to as the trabecular meshwork, is a mesh-like structure located at the iris-sclera junction in the anterior chamber of the eye. The trabeculum serves to filter aqueous fluid and control its flow from the anterior chamber into the canal of Schlemm. As the eye ages, debris and protein-lipid waste may build up and clog the trabeculum, a problem that results in increased pressure within the eye, which in turn can lead to glaucoma and damage to the retina, optic nerve, and other structures of the eye. Glaucoma drugs can help reduce this pressure, and surgery can create an artificial opening to bypass the trabeculum and reestablish flow of liquid out of the vitreous and aqueous humor. There is, however, no known method for preventing a build-up of debris and protein-lipid waste within the trabeculum.

### The iris and pupil:

With age, dilation and constriction of the iris in response to changes in illumination become slower, and its range of motion decreases. Also, the pupil becomes progressively smaller with age, severely restricting the amount of light entering the eye, especially under low light conditions. The narrowing pupil and the stiffening, slower adaptation, and constriction of the iris over time are largely responsible for the difficulty the aged have in seeing at night and adapting to changes in illumination. The changes in iris shape, stiffness, and adaptability are generally thought to come from fibrosis and cross-linking between structural proteins. Deposits of protein and lipid wastes on the iris over time may also lighten its coloration. Both the light-colored deposits on the iris, and narrowing of the pupil, are very noticeable cosmetic markers of age that may have social implications for individuals. There is no standard treatment for any of these changes, or for changes in iris coloration with age.

### The Lens:

With age, the lens yellows, becomes harder, stiffer, and less pliable, and can opacity either diffusely or in specific locations. Thus, the lens passes less light, which reduces visual contrast and acuity. Yellowing also affects color perception. Stiffening of the lens as well as the inability of the muscle to accommodate the lens results in a condition generally known as presbyopia. Presbyopia, almost always occurring after middle age, is the inability of an eye to focus correctly. This age-related ocular pathology manifests itself in a loss of accommodative ability, i.e., the capacity of the eye, through the lens, to focus on near or far objects by changing the shape of the lens to become more spherical (or convex). Both myopic and hyperopic individuals are subject to presbyopia. The age-related loss of accommodative amplitude is progressive, and presbyopia is perhaps the most prevalent of all ocular afflictions, ultimately affecting virtually all individuals during the normal human life span.

These changes in the lens are thought to be due to degenerative changes in the structure of the lens, including glycated crosslinks between collagen fibers, buildup of protein complexes, ultraviolet light degradation of structures, oxidation damage, and deposits of waste proteins, lipids, and calcium salts. Elastic and viscous properties of the lens are dependent on properties of the fiber membranes and cytoskeleton crystallins. The lens fiber membranes are characterized by an extremely high cholesterol to phospholipid ratio. Any changes in these components affect the deformability of the lens membrane. The loss of lens deformability has also been attributed to increased binding of lens proteins to the cell membranes.

Compensatory options to alleviate presbyopia currently include bifocal reading glasses and/or contact lenses, monovision intraocular lenses (IOLs) and/or contact lenses, multifocal IOLs, monovision and anisometropic corneal refractive surgical procedures using radial keratotomy (RK), photorefractive keratomileusis (PRK), and laser-assisted in situ keratomileusis (LASIK). No universally accepted treatments or cures are currently available for presbyopia.

Opacity of the lens results in an abnormal condition generally known as cataracts. Cataract formation is a progressive ocular disease, which subsequently leads to lower vision. Most of this ocular disease is age-related senile cataract. The incidence of cataract formation is thought to be 60-70% in persons in their sixties and nearly 100% in persons eighty years or older. However, at the present time, there is no agent that has been clearly proven to inhibit the development of cataracts. Therefore, the development of an effective therapeutic agent has been desired. Presently, the treatment of cataracts depends upon the correction of vision using eyeglasses, contact lenses, or surgical operations such as insertion of an intra-ocular lens into the capsula lentis after extra-capsular cataract extraction.

In cataract surgery, the incidence of secondary cataract after surgery has been a problem. Secondary cataract is equated with opacity present on the surface of the remaining posterior capsule following extracapsular cataract extraction. The mechanism of secondary cataract is mainly as follows. After excising lens epithelial cells (anterior capsule), secondary cataract results from migration and proliferation of residual lens epithelial cells, which are not completely removed at the time of extraction of the lens cortex, onto the posterior capsule leading to posterior capsule opacification. In cataract surgery, it is impossible to remove lens epithelial cells completely, and consequently it is difficult to always prevent secondary cataract. It is said that the incidence of the above posterior capsule opacification is 40-50% in eyes that do not receive an intracapsular posterior chamber lens implant and 7-20% in eyes which do receive an intracapsular lens implant. Additionally, eye infections categorized as endophthalmitis have also been observed after cataract surgeries.

### The vitreous humor:

Floaters are debris particles that interfere with clear vision by projecting shadows on the retina. There currently is no standard treatment for reducing or eliminating floaters.

### The retina:

A number of changes can occur in the retina with age. Atherosclerotic buildup and leakage in the retinal arteries can lead to macular degeneration as well as reduction of peripheral vision. The rods and cones can become less sensitive over time as they replenish their pigments more slowly. Progressively, all these effects can reduce vision, ultimately leading to partial or complete blindness. Retinal diseases such as age-related macular degeneration have been hard to cure. Current retinal treatments include laser surgery to stop the leaking of blood vessels in the eye.

As alluded to above, current therapeutic attempts to address many ocular disorders and diseases, including aging-related ocular problems, often involve surgical intervention. Surgical procedures are, of course, invasive, and, furthermore, often do not achieve the desired therapeutic goal. Additionally, surgery can be very expensive and may result in significant undesired after-effects. For example, secondary cataracts may develop after cataract surgery and infections may set in. Endophthalmitis has also been observed after cataract surgery. In addition, advanced surgical techniques are not universally available, because they require a very well developed medical infrastructure. Therefore, it would be of significant advantage to provide straightforward and effective pharmacological therapies that obviate the need for surgery.

There have been products proposed to address specific, individual aging-related ocular conditions. For example, artificial tears and herbal formulations such as Simalasan eyedrops have been suggested for treating dry eye syndrome, and other eyedrops are available to reduce intraocular pressure, alleviate discomfort, promote healing after injury, reduce inflammation, and prevent infections. However, self-administration of multiple products several times a day is inconvenient, potentially results in poor patient compliance (in turn reducing overall efficacy), and can involve detrimental interaction of formulation components. For example, the common preservative benzalkonium chloride may react with other desirable components such as ethylenediamine tetraacetic acid (EDTA). Accordingly, there is a need in the art for a comprehensive pharmaceutical formulation that can prevent, arrest, and/or reverse a multiplicity of aging-related vision problems and the associated ocular disorders.

To date, such a formulation has not been provided, in large part because complex, multicomponent pharmaceutical products are often problematic for formulators and manufacturers. Problems can arise, for example, from combining agents having different solubility profiles and/or membrane transport rates. With respect to the latter consideration, transport facilitators, also termed "permeation enhancers," often need to be incorporated into ophthalmic formulations, and must be pharmaceutically acceptable, have no effect on formulation stability, and be inert to and compatible with other components of the formulation and the physiological structures with which the formulation will come into contact.

Many adverse ocular conditions are associated with the formation, presence, and/or growth of macromolecular aggregates in the eye. Indeed, many pathologies result from or are associated with the deposition and/or aggregation of proteins, other peptidyl species, lipoproteins, lipids, polynucleotides, and other macromolecules throughout the body. For example, Advanced Glycation Endproducts (also termed AGEs) are formed by the binding of glucose or other reducing sugars to proteins, lipoproteins and DNA by a process known as non-enzymatic glycation, followed by cross-linking. These cross-linked macromolecules stiffen connective tissue and lead to tissue damage in the kidney, retina, vascular wall and nerves. AGEs have, in fact, been implicated in the pathogenesis of a variety of debilitating diseases such as diabetes, atherosclerosis, Alzheimer's and rheumatoid arthritis, as well as in the normal aging process. Peptidyl deposits are also associated with Alzheimer's disease, sickle cell anemia, multiple myeloma, and prion diseases. Lipids, particularly sterols and sterol esters, represent an additional class of biomolecules that form pathogenic deposits in - vivo, including atherosclerotic plaque, gallstones, and the like. To date, there has been no single formulation identified capable of treating a plurality of such disorders.

WO2004/058289A discloses ophthalmic formulations containing a chelating agent EDTA), an ophtalmic permeation enhancer (MSM), an active agent (anti-AGE agent : L-carnosine) and de-ionized water to prevent/treat from adverse ocular conditions, for instance presbyopia or arcus senilis.

US-A-5817630 discloses an ophthalmic composition comprising an heavy metal chelator (EDTA), an antioxidant (vitamin E), a penetrant (DMSO), ascorbic acid, L-glutathione. The ingredients are diluted in a suitable diluent and administered to patient as eye drop to decrease the effects of presbyopia DMSO is used as penetrant and can be replaced by DMSO₂ (i.e MSM) in the formulation.

US2004/137068A discloses formulations comprising a chelating agent (EDTA), MSM, water and L-carnosine and for the treatment of ocular conditions associated with aging. The chelating agent can be a chelating antibiotic, for instance a tetracyclin.

Unit Ubeyt l. et. al. J. Catas. Ref. Surg. 27(7) 2001 p1079-1087 discloses EDTA, mitomycin C, diclofenac or dexamethasone as pharmacological mean to prevent and reduce the development of capsule opacification.

WO2004/087043A discloses ophthalmic compositions comprising an anti infective agent (ciprofloxacin), a steroidal anti-inflammatory agent (dexamethasone), a chelating agent (EDTA) in a liquid vehicle (water).

### DISCLOSURE OF THE INVENTION

The present invention is directed to the aforementioned need in the art.

According to one aspect the present invention provides use of a permeation enhancing amount of methylsulfonylmethane for the preparation of a formulation containing ciprofloxacin, and a pharmaceutically acceptable ophthalmic carrier, for delivering ciprofloxacin to the eye of a patient, wherein the permeation enhancing amount of methylsulfonylmethane is in the range of 1.0 wt.% to 33 wt.% of the formulation.

According to another aspect the present invention provides a formulation for delivering an ophthalmologically active agent to the eye of a patient, the formulation characterized in that the formulation contains, in addition to an effective amount of an ophthalmologically active agent, a pharmaceutically acceptable ophthalmic carrier, a permeation enhancing amount of methylsulfonylmethane in the range of 1.0 wt.% to 33 wt.% of the formulation, wherein the ophthalmologically active agent is ciprofloxacin.

The adverse ocular conditions which may be treated by the use or formulation include, by way of example, conditions, diseases, or disorders of the cornea, retina, lens, sclera, and anterior and posterior segments of the eye.

Thus the invention pertains to the use of methysulfonylmethane as a transport enhancer (i.e., a "permeation enhancer") for the delivery of ciprofloxacin. A sterile ophthalmic formulation is provided for the treatment of an ophthalmic condition comprising methylsulfonylmethane and a therapeutically effective amount of a pharmacologically active agent, i.e., an ophthalmologically active agent, in a pharmaceutically acceptable carrier. We describe that methylsulfonylmethane can be administered to the eye in the absence of an added ophthalmologically active agent.

### BRIEF DESCRIPTION OF THE FIGURES

FIGS. 1A, 1B, 2A, and 2B are photographs of the eyes of a 46-year-old male subject prior to treatment (OD - FIG. 1A; OS-FIG. 2A) and after (OS - FIG. 1B; and OS-FIG. 2B) receiving eight weeks of treatment with an eye drop formulation of the invention, as described in Example 5.

FIGS. 3A, 3B, 4A, and 4B are photographs of the eyes of a 60-year-old male subject prior to treatment (OD - FIG. 3A; OS-FIG. 4A) and after (OS - FIG. 3B; and OS-FIG. 3B) receiving eight weeks of treatment with an eye drop formulation of the invention, as described in Example 6.

FIG. 5 compares the contrast sensitivity improvement resulting from Formulation 3 compared to placebo in Example 14.

FIG. 6 compares the penetration of solutions A, B, and C in Example 15 after 30 minutes, 2 hours, and 16 hours.

FIGS. 7A and 7B depict the permeation of EDTA as found in Example 16.

FIGS. 8A and 8B depict the effect of various treatments from Example 17.

FIG. 9 depicts the transmission in rat lenses as a function of treatment in Example 17.

FIG. 10 depicts the effect of various treatments on cell viability as found in Example 18.

FIG. 11 depicts the permeation results of Example 18.

FIG. 12 depicts the permeation results of Example 19.

FIG. 13 depicts the cumulative permeation of ciprofloxacin through porcine intestinal membrane as measured in Example 20.

FIG. 14 depicts the cumulative permeation of ciprofloxacin-HCl through porcine intestinal membrane as measured in Example 21.

FIG. 15 depicts cumulative permeation of ciprofloxacin-HCl through porcine intestinal membrane as measured in Example 22.

FIG. 16 depicts the cumulative permeation of Methylene Blue though pre-treated porcine intestinal membrane as measured in Example 27.

### DETAILED DESCRIPTION OF EMBODIMENTS OF THE INVENTION

Unless otherwise indicated, the invention is not limited to specific formulation types, formulation components, dosage regimens, or the like, as such may vary. It is also to be understood that the terminology used herein is for the purpose of describing particular embodiments only and is not intended to be limiting.

As used in the specification and the appended claims, the singular forms "a," "an," and "the" include plural referents unless the context clearly dictates otherwise. Thus, for example, reference to "a chelating agent" includes a single such agent as well as a combination or mixture of two or more different chelating agents, reference to "a permeation enhancer" includes not only a single permeation enhancer but also a combination or mixture of two or more different permeation enhancers, reference to "a pharmaceutically acceptable vehicle" includes two or more such vehicles as well as a single vehicle, and the like.

In this specification and in the claims that follow, reference will be made to a number of terms, which shall be defined to have the following meanings:

When referring to a formulation component, it is intended that the term used, e.g., "agent" or "component," encompass not only the specified molecular entity but also its pharmaceutically acceptable analogs, including, but not limited to, salts, esters, amides, prodrugs, conjugates, active metabolites, and other such derivatives, analogs, and related compounds.

The terms "treating" and "treatment" as used herein refer to the administration of an agent or formulation to a clinically symptomatic individual afflicted with an adverse condition, disorder, or disease, so as to effect a reduction in severity and/or frequency of symptoms, eliminate the symptoms and/or their underlying cause, and/or facilitate improvement or remediation of damage. The terms "preventing" and "prevention" refer to the administration of an agent or composition to a clinically asymptomatic individual who is susceptible to a particular adverse condition, disorder, or disease, and thus relates to the prevention of the occurrence of symptoms and/or their underlying cause. Unless otherwise indicated herein, either explicitly or by implication, if the term "treatment" (or "treating") is used without reference to possible prevention, it is intended that prevention be encompassed as well, such that "a method for the treatment of presbyopia" would be interpreted as encompassing "a method for the prevention of presbyopia."

By the terms "effective amount" and "therapeutically effective amount" of a formulation or formulation component is meant a nontoxic but sufficient amount of the formulation or component to provide the desired effect.

The term "controlled release" refers to an agent-containing formulation or fraction thereof in which release of the agent is not immediate, i.e., with a "controlled release" formulation, administration does not result in immediate release of the agent into an absorption pool. The term is used interchangeably with "nonimmediate release" as defined in Remington: The Science and Practice of Pharmacy, Nineteenth Ed. (Easton, PA: Mack Publishing Company, 1995). In general, the term "controlled release" as used herein refers to "sustained release" rather than to "delayed release" formulations. The term "sustained release" (synonymous with "extended release") is used in its conventional sense to refer to a formulation that provides for gradual release of an agent over an extended period of time.

By a "pharmaceutically acceptable" or "ophthalmologically acceptable" component is meant a component that is not biologically or otherwise undesirable, i.e., the component may be incorporated into an ophthalmic formulation of the invention and administered topically to a patient's eye without causing any undesirable biological effects or interacting in a deleterious manner with any of the other components of the formulation composition in which it is contained. When the term "pharmaceutically acceptable" is used to refer to a component other than a pharmacologically active agent, it is implied that the component has met the required standards of toxicological and manufacturing testing or that it is included on the Inactive Ingredient Guide prepared by the U.S. Food and Drug Administration.

The phrase "having the formula" or "having the structure" is not intended to be limiting and is used in the same way that the term "comprising" is commonly used.

The amount of the transport enhancer in the present formulation is an effective enhancing amount; that is, the transport enhancer is present at a concentration sufficient to provide a measurable increase in penetration of one or more of the formulation components through membranes, tissues, and extracellular matrices as just described. The permeation enhancer is methylsulfonylmethane (MSM; also referred to as methyl sulfone), combinations of MSM with dimethylsulfoxide (DMSO), or a combination of MSM and, in a less preferred embodiment, DMSO, with MSM particularly preferred.

MSM is an odorless, highly water-soluble (34% w/v @ 79 °F) white crystalline compound with a melting point of 108-110 °C and a molecular weight of 94.1 g/mol. MSM serves as a multifunctional agent herein, insofar as the agent not only increases cell membrane permeability, but also acts as a "transport facilitating agent" (TFA) that aids in the transport of one or more formulation components to both the anterior and posterior of the eye. Furthermore, MSM *per se* provides medicative effects, and can serve as an anti-inflammatory agent as well as an analgesic. MSM also acts to improve oxidative metabolism in biological tissues, and is a source of organic sulfur, which assists in the reduction of scarring. MSM additionally possesses unique and beneficial solubilization properties, in that it is soluble in water, as noted above, but exhibit both hydrophilic and hydrophobic properties because of the presence of polar S=O groups and nonpolar methyl groups. The molecular structure of MSM also allows for hydrogen bonding with other molecules, i.e., between the oxygen atom of each S=O group and hydrogen atoms of other molecules, and for formation of van der Waals associations, i.e., between the methyl groups and nonpolar (e.g., hydrocarbyl) segments of other molecules. The concentration of MSM in the present formulations is in the range of about 1.0 wt.% to 33 wt.%, preferably about 1.5 wt.% to 8.0 wt.%.

The formulation can also include a microcirculatory enhancer, i.e., an agent that serves to enhance blood flow within the capillaries. The microcirculatory enhancer can be a phosphodiesterase (PDE) inhibitor, for instance a Type (I) PDE inhibitors. Such compounds, as will be appreciated by those of ordinary skill in the art, act to elevate intracellular levels of cyclic AMP (cAMP). A preferred microcirculatory enhancer is vinpocetine, also referred to as ethyl apovincamin-22-oate. Vinpocetine, a synthetic derivative of vincamine, a Vinca alkaloid, is particularly preferred herein because of its antioxidant properties and protection against excess calcium accumulation in cells. Vincamine is also useful as a microcirculatory enhancer herein, as are Vinca alkaloids other than vinpocetine. Preferably, any microcirculatory enhancer present, e.g., vinpocetine, represents about 0.01 wt.% to about 0.2 wt.%, preferably in the range of about 0.02 wt.% to about 0.1 wt.% of the formulation.

Other optional additives in the present formulations include secondary enhancers, i.e., one or more additional permeation enhancers. For example, formulation of the invention can contain added dimethylsulfoxide (DMSO). If DMSO is added as a secondary enhancer, the amount is preferably in the range of about 1.0 wt.% to 2.0 wt.% of the formulation, and the weight ratio of MSM to DMSO is typically in the range of about 1:1 to about 50:1. or example, formulation of the invention can contain added DMSO. As discussed above, methylsulfonylmethane (MSM) has particular utility as a penetration enhancer in ophthalmic applications. The invention describes a sterile ophthalmic formulation for the treatment of an ophthalmic condition comprising MSM and an amount of an active ingredient effective for the treatment of that condition in a pharmaceutically acceptable carrier. In a preferred version of this embodiment, the formulation consists essentially of MSM and the active ingredient.

Other possible additives for incorporation into the formulations that are at least partially aqueous include, without limitation, thickeners, isotonic agents, buffering agents, and preservatives, providing that any such excipients do not interact in an adverse manner with any of the formulation's other components. Suitable thickeners will be known to those of ordinary skill in the art of ophthalmic formulation, and include, by way of example, cellulosic polymers such as methylcellulose (MC), hydroxyethylcellulose (HEC), hydroxypropylcellulose (HPC), hydroxypropyl-methylcellulose (HPMC), and sodium carboxymethylcellulose (NaCMC), and other swellable hydrophilic polymers such as polyvinyl alcohol (PVA), hyaluronic acid or a salt thereof (e.g., sodium hyaluronate), and crosslinked acrylic acid polymers commonly referred to as "carbomers" (and available from B.F. Goodrich as Carbopol® polymers). The preferred amount of any thickener is such that a viscosity in the range of about 15 cps to 25 cps is provided, as a solution having a viscosity in the aforementioned range is generally considered optimal for both comfort and retention of the formulation in the eye. Any suitable isotonic agents and buffering agents commonly used in ophthalmic formulations may be used, providing that the osmotic pressure of the solution does not deviate from that of lachrymal fluid by more than 2-3% and that the pH of the formulation is maintained in the range of about 6.5 to about 8.0, preferably in the range of about 6.8 to about 7.8, and optimally at a pH of about 7.4. Preferred buffering agents include carbonates such as sodium and potassium bicarbonate.

The formulations of the invention also include a pharmaceutically acceptable ophthalmic carrier or vehicle, which will depend on the particular type of formulation. For example, the formulations of the invention can be provided as an ophthalmic solution or suspension, in which case the carrier is at least partially aqueous. Ideally, ophthalmic solutions, which may be administered as eye drops, are aqueous solutions. The formulations may also be ointments, in which case the pharmaceutically acceptable carrier is composed of an ointment base. Preferred ointment bases herein have a melting or softening point close to body temperature, and any ointment bases commonly used in ophthalmic preparations may be advantageously employed. Common ointment bases include petrolatum and mixtures of petrolatum and mineral oil.

The ophthalmologically active agent that is incorporated into the present formulation is ciprofloxacin.

The formulations of the invention may be prepared as a hydrogel, dispersion, or colloidal suspension. Hydrogels are formed by incorporation of a swellable, gel-forming polymer such as those set forth above as suitable thickening agents (i.e., MC, HEC, HPC, HPMC, NaCMC, PVA, or hyaluronic acid or a salt thereof, e.g., sodium hyaluronate), except that a formulation referred to in the art as a "hydrogel" typically has a higher viscosity than a formulation referred to as a "thickened" solution or suspension. In contrast to such preformed hydrogels, a formulation may also be prepared so as to form a hydrogel in situ following application to the eye. Such gels are liquid at room temperature but gel at higher temperatures (and thus termed "thermoreversible" hydrogels), such as when placed in contact with body fluids. Biocompatible polymers that impart this property include acrylic acid polymers and copolymers, N-isopropylacrylamide derivatives, and ABA block copolymers of ethylene oxide and propylene oxide (conventionally referred to as "poloxamers" and available under the Pluronic® tradename from BASF-Wyandotte). The formulations can also be prepared in the form of a dispersion or colloidal suspension. Preferred dispersions are liposomal, in which case the formulation is enclosed within "liposomes," microscopic vesicles composed of alternating aqueous compartments and lipid bilayers. Colloidal suspensions are generally formed from microparticles, i.e., from microspheres, nanospheres, microcapsules, or nanocapsules, wherein microspheres and nanospheres are generally monolithic particles of a polymer matrix in which the formulation is trapped, adsorbed, or otherwise contained, while with microcapsules and nanocapsules, the formulation is actually encapsulated. The upper limit for the size for these microparticles is about 5 µm to about 10 µm.

The use and formulations of the invention are useful in treating a host of adverse ocular conditions, including conditions, diseases or disorders of the cornea, retina, lens, sclera, and anterior and posterior segments of the eye, many of which involve the formation or deposition of molecular aggregates as discussed above. Of particular interest are those adverse ocular conditions associated with the aging process and/or oxidative and free radical damage to the eye. It should be emphasized, however, that the adverse condition treated may or may not be age-related, and the invention includes methods of treating both types of conditions.

### Reference EXAMPLE 1

An eye drop formulation of the invention, Formulation **1,** was prepared as follows: High purity de-ionized (DI) water (500 ml) was filtered via a 0.2 micrometer filter. MSM (27 g), EDTA (13 g), and L-carnosine (5 g) were added to the filtered DI water, and mixed until visual transparency was achieved, indicating dissolution. The mixture was poured into 10 mL bottles each having a dropper cap. On a weight percent basis, the eye drops had the following composition:

| | |
|---|---|
| Purified de-ionized water | 91.74 wt.% |
| MSM | 4.95 wt.% |
| Di-sodium EDTA | 2.39 wt.% |
| L-Carnosine | 0.92 wt.%. |

### Reference EXAMPLE 2

Formulation **1** was evaluated for efficacy in treating four subjects, all males between 52 and 84 years of age of mixed ethnicity. Subject 1 was in his fifties and had no visual problems or detectable abnormalities of the eye. Subjects 2 and 3 were in their fifties and had prominent arcus senilis around the cornea periphery in both eyes but no other adverse ocular conditions (arcus senilis is typically considered to be a cosmetic blemish). Subject 4 was in his eighties and was suffering from cataracts and Salzmann's nodules, and reported extreme photophobia and problems with glare. This subject was having great difficulty reading newspapers, books, and information on a computer screen, because of the glare and loss in visual clarity.

The formulation was administered to the subjects, one drop (approximately 0.04 mL) to each eye, two to four times per day for a period of over 12 months. All subjects were examined by an ophthalmologist during and after 12 months. No side effects, other than minor temporary irritation at the time of administering the formulation in the eye, were reported or observed by the subjects or the ophthalmologist. All four subjects completed the study.

All subjects noticed subjective changes 4 weeks into the study. At this stage, the changes reported by the subjects included increased brightness, improved clarity of vision, and reduced glare (particularly Subject 4).

After 8 weeks, the following changes were noted: All four subjects reported greatly improved vision with regard to clarity and contrast, and indicated that daytime colors appeared to increase in brilliance. Subject 1's eyesight improved from 20/25 (after correction) to better than 20/20 (with the same correction), and his eyes turned a deeper shade of blue. Subjects 2 and 3 exhibited a significant reduction of the arcus senilis.

For Subject 4, whose vision originally with best correction had been 20/400 in his left eye and 20/200 in his right eye and had acute photophobia and glare. The glare and photophobia were reduced, and the subject started to read books, newspapers, and information on the computer screen again. The visual acuity in his right eye improved significantly, from 20/200 (with correction) to 20/60 (pinhole) (with the same correction). In his left eye, his visual acuity improved as well, from 20/400 to 20/200 (with the same correction). In his left eye, he continued to have a central dark spot due to macular scarring.

After 16 weeks, the following changes were noted: All subjects reported continuing improvement of vision, including night vision, as well as improved contrast sensitivity and continued improvement in color perception. Subject 1's eyesight continued to improve, from 20/20 (after correction) to 20/15 (witch the same correction). Subjects 2 and 3 continued to exhibit a reduction of the arcus senilis.

Subject 4 reported a further reduction in glare and photophobia, and further improvements in the ease of reading books, newspapers, and information on the computer screen. Subject 4 also reported that nighttime glare had been eliminated. The subject was now comfortable in daylight without need for dark glasses, and without suffering severe problems with glare. The visual acuity in his right eye improved from 20/60 (pinhole) to 20/50 (pinhole). In his left eye his visual acuity also improved, from 20/200 to 20/160 (with same correction). In his left eye, he continued to have a central dark spot due to macular scarring.

After eight months, Subject 4's vision in his right eye improved from 20/50 (pinhole) to 20/40 (pinhole) In his left eye his visual acuity improved from 20/160 to 20/100 (with same correction). The dark spot in the left eye started dissipating, and he could read hazily through the formerly dark spot. At this time his contrast sensitivity was also measured. His cataracts were measured at a 4+ (on a scale of 0-4, 4 being the highest). The central macular scar was barely visible to the ophthalmologist due to haziness of the optical path. After 10 months, Subject 1's visual acuity further improved from 20/15 to 20/10 (with the same correction).

After further 2 months; i.e., after a total of 12 months, Subject 4's vision continued to improve. The subject could now read books, newspapers, and the computer screen without any problems. The subject also showed improvement in cataracts (went from 4+ to 3-4+ on a 0-4 scale). The optical path clarity had improved enough that the macular scar was clearly visible to the ophthalmologist. In contrast sensitivity there was a 40% to 100% improvement. In Snellen acuity, he went from 20/40 to 20/30 (pinhole) in his right eye, and from 20/100 to 20/80 in his left eye.

### Reference EXAMPLE 3

A second eye drop formulation of the invention, Formulation 2, was prepared as follows: High purity de-ionized (DI) water (500 ml) was filtered via a 0.2 micrometer filter. MSM (13.5 g), EDTA (6.5 g), and L-carnosine (5.0 g) were added to the filtered DI water, and mixed until visual transparency was achieved, indicating dissolution. The mixture was poured into 10 mL bottles each having a dropper cap. On a weight percent basis, the eye drop composition had the following components:

| | |
|---|---|
| Purified de-ionized water | 95.24 wt.% |
| MSM | 2.57 wt.% |
| Di-sodium EDTA | 1.24 wt.% |
| L-Carnosine | 0.95 wt.% |

### Reference EXAMPLE 4

Subsequent to the experimentation described in Reference Example 2, a detailed and controlled follow-on study was carried out using a slightly weaker eye drop formulation, Formulation 2 (prepared as described in Reference Example 3). Placebo eye drops were also prepared and administered. The placebo drops were composed of a commercially obtained sterile saline solution in the form of a buffered isotonic aqueous solution (containing boric acid, sodium borate, and sodium chloride with 0.1 wt.% sorbic acid and 0.025 wt.% di-sodium EDTA as preservatives).

The study was double-masked, in that except for one positive control, neither the patient nor the ophthalmologist knew whether they were given the formulation eye drops or a saline solution. The patients were randomized to receive either the study formulation or saline solution.

The study involved five subjects, of which 3 subjects were given the eye drops of Formulation 2 and 1 subject was given placebo eye drops. In addition, 1 subject was given the higher-strength eye drops of Formulation 1. One drop (approximately 0.04 mL) was administered to each eye, two to four times daily for a period of 8 weeks. The drops were administered to both eyes of each subject. The study participants were multiethnic and 20% female, 80% male.

The baseline and follow-on testing by the ophthalmologist included: automated refraction; corneal topography; external photographs; wavefront photographs; visual acuity with spectacle correction at distance and at 14 inches; contrast sensitivity testing using the Vision Sciences Research Corporation (San Ramon, California) Functional Acuity Contrast Test (FACT) chart; pupil examination and pupil size measurement; slit lamp examination; intraocular pressure measurement; and dilated fundus examination.

After 8 weeks, the subjects were examined again. The contrast sensitivity results for each subject are shown in Table 1, and all the results are summarized in Table 2.

| Table 1 | | | | | | | | | | | |
|---|---|---|---|---|---|---|---|---|---|---|---|
| Subject | | 1 | | 2 | | 3 | | 4⁵ | | 5⁶ | |
| Right (R) or Left (L) Eye | | R | L | R | L | R | L | R | L | R | L |
| Contrast Sensitivity (CS)¹ | | | | | | | | | | | |
| | | | | | | | | | | | |
| 1.5 cpd² | log₁₀ CS before | 1.85 | 1.56 | 1.70 | 1.70 | 2.00 | 1.85 | 1.56 | 1.70 | 1.85 | 1.70 |
| | log₁₀ CS after | 2.00 | 1.85 | 1.85 | 1.85 | 2.00 | 1.85 | 1.85 | 1.85 | 1.85 | 1.56 |
| | log₁₀ unit change³ | 0.15 | 0.29 | 0.15 | 0.15 | 0.00 | 0.00 | 0.29 | 0.15 | 0.00 | -.14 |
| | percent improved⁴ | 8 | 19 | 9 | 9 | 0 | 0 | 19 | 9 | 0 | -8 |
| | | | | | | | | | | | |
| 3 cpd | log₁₀ CS before | 1.90 | 1.76 | 1.90 | 1.90 | 1.90 | 1.90 | 1.76 | 1.90 | 1.76 | 1.90 |
| | log₁₀ CS after | 2.06 | 1.90 | 1.90 | 2.06 | 2.06 | 2.06 | 2.20 | 2.06 | 1.90 | 1.76 |
| | log₁₀ unit change | 0.16 | 0.14 | 0.00 | 0.16 | 0.16 | 0.16 | 0.44 | 0.16 | 0.14 | -.14 |
| | percent improved | 8 | 8 | 0 | 8 | 8 | 8 | 26 | 8 | 8 | -8 |
| | | | | | | | | | | | |
| 6 cpd | log₁₀ CS before | 1.81 | 1.81 | 1.95 | 2.11 | 1.95 | 1.95 | 1.65 | 1.81 | 1.81 | 1.81 |
| | log₁₀ CS after | 1.95 | 1.81 | 2.11 | 1.95 | 2.11 | 2.11 | 2.11 | 2.11 | 1.81 | 1.95 |
| | log₁₀ unit change | 0.14 | 0.00 | 0.16 | -.16 | 0.16 | 0.16 | 0.46 | 0.30 | 0.00 | 0.14 |
| | percent improved | 8 | 0 | 8 | -7 | 8 | 8 | 27 | 17 | 0 | 8 |
| | | | | | | | | | | | |
| 12 cpd | log₁₀ CS before | 1.34 | 1.18 | 1.78 | 1.78 | 1.78 | 1.78 | 1.18 | 1.48 | 1.48 | 1.63 |
| | log₁₀ CS after | 1.63 | 1.48 | 1.78 | 1.78 | 1.78 | 1.78 | 1.93 | 1.78 | 1.63 | 1.48 |
| | log₁₀ unit change | 0.29 | 0.30 | 0.00 | 0.00 | 0.00 | 0.00 | 0.75 | 0.30 | 0.15 | -.15 |
| | percent improved | 22 | 26 | 0 | 0 | 0 | 0 | 64 | 20 | 11 | -10 |
| | | | | | | | | | | | |
| 18 cpd | log₁₀ CS before | 0.90 | 1.08 | 1.23 | 1.23 | 1.23 | 1.30 | 0.60 | 1.23 | 0.90 | 1.23 |
| | log₁₀ CS after | 1.36 | 1.36 | 1.36 | 1.36 | 1.52 | 1.52 | 1.66 | 1.52 | 1.36 | 1.36 |
| | log₁₀ unit change | 0.46 | 0.28 | 0.13 | 0.13 | 0.29 | 0.22 | 1.06 | 0.29 | 0.46 | 0.13 |
| | **PERCENT IMPROVED** | 51 | 27 | 11 | 11 | 23 | 12 | 176 | 23 | 51 | 11 |

| | | | | | | | | | | | |
|---|---|---|---|---|---|---|---|---|---|---|---|
| ¹Contrast sensitivity (CS) is the reciprocal of the contrast at threshold, i.e., one divided by the lowest contrast at which forms or lines can be recognized. Log of the contrast sensitivity values is a generally accepted method for comparing contrast sensitivities. ²cpd = cycles per degree for the spatial frequency ³Log unit change = log₁₀(CS after treatment) - log₁₀(CS before treatment) ⁴Percent improved = [log₁₀(CS after treatment)/log₁₀(CS before treatment)-1] × 100 ⁵Positive control ⁶Placebo | | | | | | | | | | | |

| Table 2 | | | | |
|---|---|---|---|---|
| | | Formulation **1** (positive control) n=1 | Formulation **2** (study subjects) n=3 | Saline Solution (placebo) n=1 |
| **PUPIL DILATION** | | +20% | +8% | 0% |
| Snellen Acuity (distance vision) | | +17.5% | +7.5% | -15% |
| Snellen Acuity (near vision) | | 0% | +10% | 0% |
| Auto refraction | | +8% | +8% | 0% |
| Contrast Sensitivity¹ | | | | |
| | | | | |
| 1.5 cpd² | percent improved³ | 14% | 7.5% | -4% |
| | log unit change⁴ | 0.22 | 0.12 | -0.08 |
| | | | | |
| 3 cpd | percent improved | 17% | 6.8% | 0% |
| | log unit change | 0.33 | 0.12 | 0 |
| | | | | |
| 6 cpd | percent improved | 22% | 4% | 4% |
| | log unit change | 0.38 | 0.08 | 0.08 |
| | | | | |
| 12 cpd | percent improved | 42% | 7.9% | 0% |
| | log unit change | 0.53 | 0.10 | 0 |
| | | | | |
| 18 cpd | percent improved | 99.5% | 22.2% | 31.0% |
| | log unit change | 0.68 | 0.24 | 0.26 |
| Wavefront (image tightness) | | +23% | +38% | 0% |

| | | | | |
|---|---|---|---|---|
| ¹Contrast sensitivity (CS) is the reciprocal of the contrast at threshold, i.e., one divided by the lowest contrast at which forms or lines can be recognized. Log of the contrast sensitivity values is a generally accepted method for comparing contrast sensitivities. ²cpd = cycles per degree for the spatial frequency ³Percent improved = [log₁₀(CS after treatment)/log₁₀(CS before treatment)-1] × 100 ⁴Log unit change = log₁₀(CS after treatment) - log₁₀(CS before treatment) | | | | |

Subjects treated with Formulation 1 and Formulation 2 all showed very significant improvements, including improved smoothness and regularity of the cornea, improved accommodative/focusing ability, more uniform and stable tear film, and decreased yellowing of the cornea and lens. Subjects to whom the placebo was given did not exhibit any significant change. All subjects reported improved ability to see road signs at a distance, brighter and more vivid colors, and improved night vision.

### Reference EXAMPLE 5

Formulation 1 was evaluated for efficacy in a 46-year-old male subject. Prior to treatment, the subject had no severe visual problems or eye abnormalities, but he did require bifocals to correct refractive errors in both eyes.

The subject was examined by an independent ophthalmologist prior to treatment and again following eight weeks of treatment. Tests performed included: Snellen visual acuity examinations for distance (20 feet) and near (14 inches) vision, autorefraction, pupil dilation (pupillometer maximum scotopic pupil size), slit lamp examination, automated corneal topography mapping, contrast sensitivity (functional acuity contrast test), automated wavefront aberration mapping, and photographs of the anterior segment.

Treatment consisted of the topical instillation of one drop (approximately 0.04 mL) of Formulation **1** in each eye two to four times per day for eight weeks. Results of this treatment were as follows:

No irritation, redness, pain, or other adverse effects were observed by the ophthalmologist or reported by the subject, other than transient minor eye irritation at the time of eye drop administration.

Snellen visual acuity: Using the same refractive correction, distance visual acuity improved from 20/25+1 to 20/20 in the right eye, and from 20/20-2 to 20/20 in the left eye. Near vision was unchanged at 20/50 in both eyes.

Autorefraction: The right eye was unchanged: spherical -3.75; astigmatism +2.5 at axis of 24 degrees. The left eye showed slight improvement: spherical decreased from -4.00 to -3.75; astigmatism decreased from +3.50 at 175 degrees to +3.25 at 179 degrees.

Pupil dilation: Both eyes improved from 5.0 to 6.0 mm.

Slit lamp examination: The retinas appeared unchanged, and no cataracts were observed during either examination.

Corneal topography: Improved smoothness and regularity of the cornea were observed in both eyes. The ophthalmologist remarked that the improvement may have been due to a more uniform and stable tear film.

Contrast sensitivity: Measurements are shown in Table 3.

| Table 3 | | | | | | | | | | |
|---|---|---|---|---|---|---|---|---|---|---|
| CPD* | 1.5 | | 3 | | 6 | | 12 | | 18 | |
| Eye | R | L | R | L | R | L | R | L | R | L |
| Before | 6 | 7 | 6 | 7 | 5 | 6 | 3 | 5 | 1 | 5 |
| After | 8 | 8 | 9 | 8 | 8 | 8 | 8 | 7 | 8 | 7 |

| | | | | | | | | | | |
|---|---|---|---|---|---|---|---|---|---|---|
| *CPD = cycles per degree (spatial frequency of pattern) | | | | | | | | | | |

These data indicate a consistent, significant improvement in contrast sensitivity.

Automated wavefront mapping. For the right eye, spherical aberration was essentially unchanged (+0.15660 to +0.15995). Retinal image formation improved from 60x70 to 45x70 minutes of arc, which represents a 25% tighter image formation. For the left eye: Spherical aberration decreased from +0.14512 to +0.09509, representing a 34.4% improvement. Retinal image formation improved with an estimated 20% tighter image.

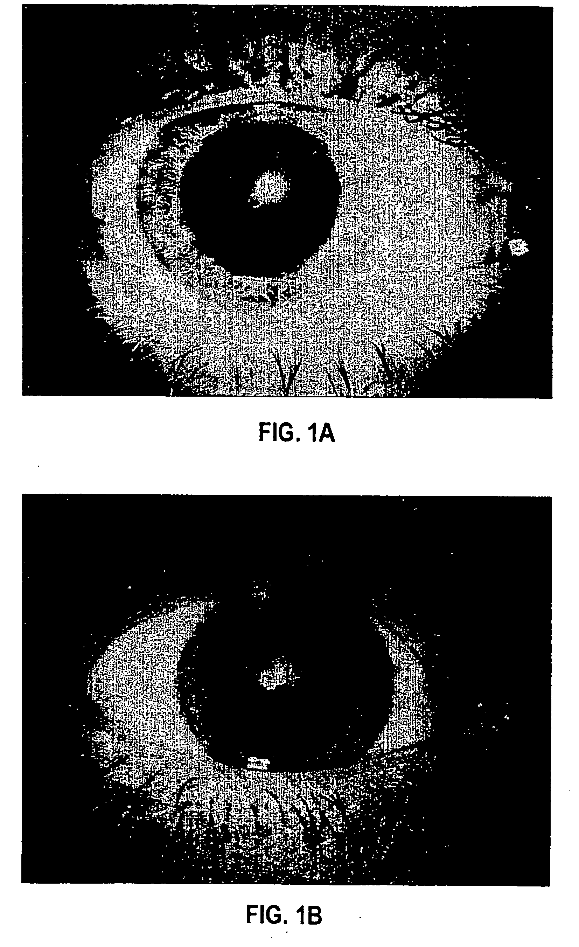

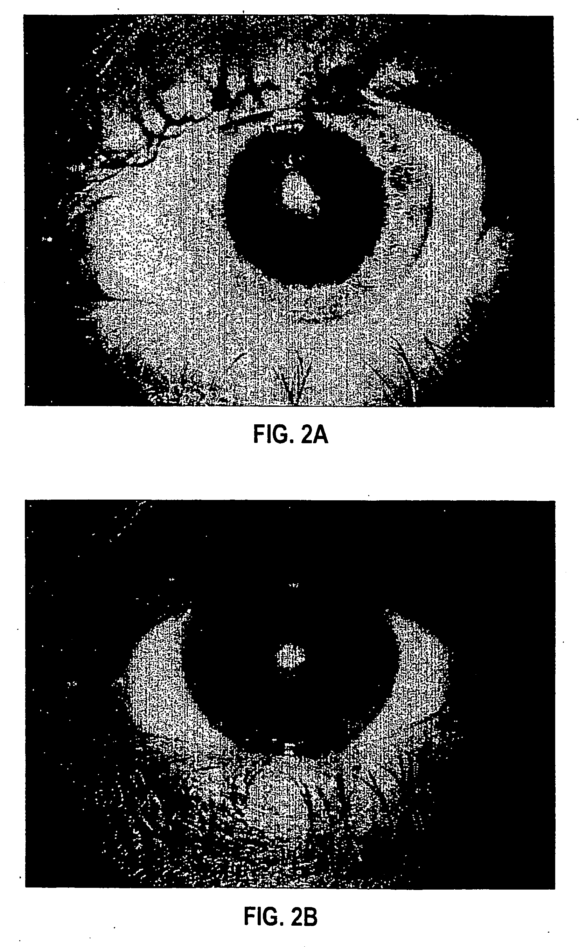

Photographs of anterior segment, FIG. 1A (OD, before treatment), FIG. 1B (OD, after treatment), FIG. 2A (OS, before treatment), and FIG. 2B (OS, after treatment): Iris color changed to a darker blue; the degree of change was reported as "striking." The change was likely due to a decrease in the yellowing of the cornea.

In addition, the subject reported that, following treatment, he switched to lower power prescription glasses and no longer required bifocals. He made the following remarks: "I have been using the eye drops for about eight weeks, and my eyesight has significantly improved. I can see colors more vividly. I have replaced my bifocals with my older, lower power non-bifocals. I can see much better in the distance and do not need reading glasses. My eyes have become a darker blue like my original eye color, and my night vision has improved."

### Reference EXAMPLE 6

Formulation 1 was evaluated for efficacy in a 60-year-old male subject. Prior to treatment, the subject had no serious visual problems or eye abnormalities other than refractive errors in both eyes.

The subject was examined by an independent ophthalmologist prior to treatment and again following seven weeks of treatment. Tests performed included: Snellen visual acuity examinations for distance (20 feet) and near (14 inches) vision, autorefraction, pupil dilation (pupillometer maximum scotopic pupil size), slit lamp examination, automated corneal topography mapping, contrast sensitivity (functional acuity contrast test), automated wavefront aberration mapping, and photographs of the anterior segment.

Treatment consisted of the topical instillation of one drop (approximately 0.04 mL) of Formulation 1 in each eye two to four times per day for seven weeks. Results of this treatment were as follows:

No irritation, redness, pain, or other adverse effects were observed by the ophthalmologist or reported by the subject, other than transient minor eye irritation at the time of eye drop administration.

Snellen visual acuity: Using the same refractive correction (intentionally undercorrected in the left eye), distance visual acuity remained unchanged at 20/15 in the right eye, and improved from 20/40-2 to 20/40 in the left eye. Near vision declined from 20/70 to 20/100 in the right eye (likely due to overcorrection for distance), and improved from 20/40-2 to 20/25 in the left eye.

Autorefraction: The right eye had an unchanged spherical measurement

(-6.00) and a slight improvement in astigmatism (+0.75 at 115 degrees to +0.50 at 113 degrees). The left eye showed slight improvement: spherical went from -8.25 to -8.00; astigmatism was unchanged, from +1.00 at 84 degrees to +1.00 at 82 degrees.

Pupil dilation: The right eye improved from 4.0 to 4.5 mm, and the left eye was unchanged at 4.0 mm.

Slit lamp examination: The retinas appeared unchanged, and minimal cataracts were observed during both examinations.

Corneal topography: Improved smoothness and regularity of the cornea were observed in both eyes. The ophthalmologist remarked that the improvement may have been due to a more uniform and stable tear film.

Contrast sensitivity: Measurements are shown in Table 4.

| Table 4 | | | | | | | | | | |
|---|---|---|---|---|---|---|---|---|---|---|
| CPD* | 1.5 | | 3 | | 6 | | 12 | | 18 | |
| Eye | R | L | R | L | R | L | R | L | R | L |
| Before | 8 | 6 | 7 | 6 | 6 | 6 | 4 | 3 | 3 | 4 |
| After | 9 | 8 | 8 | 7 | 7 | 6 | 6 | 5 | 6 | 6 |

| | | | | | | | | | | |
|---|---|---|---|---|---|---|---|---|---|---|
| *CPD = cycles per degree (spatial frequency of pattern) | | | | | | | | | | |

These data indicate a consistent, significant improvement in contrast sensitivity.

Automated wavefront mapping: For the right eye: Spherical aberration decreased from +0.01367 to +0.00425, a 69% improvement. Retinal image formation improved from 80x80 to 70x65 minutes of arc, which represents a 28.9% tighter image formation. For the left eye: Spherical aberration decreased from +0.04687 to -0.00494, representing a >100% improvement. Retinal image formation improved from 150x150 to 100x100 minutes of arc, which represents a 33% tighter image formation. The ophthalmologist remarked at the second examination: "Overall spherical aberration is closer to that of a young healthy eye rather than a 60-year-old eye."

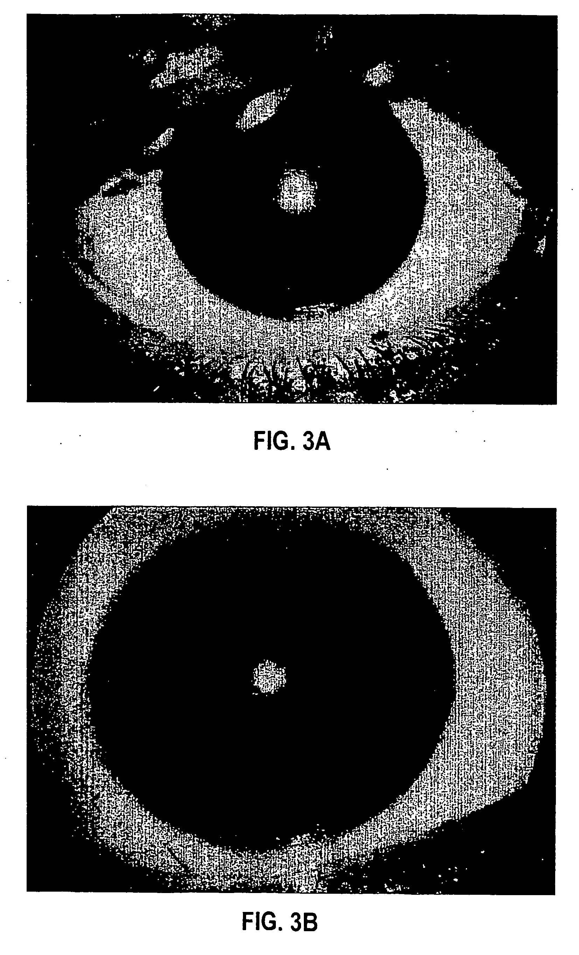

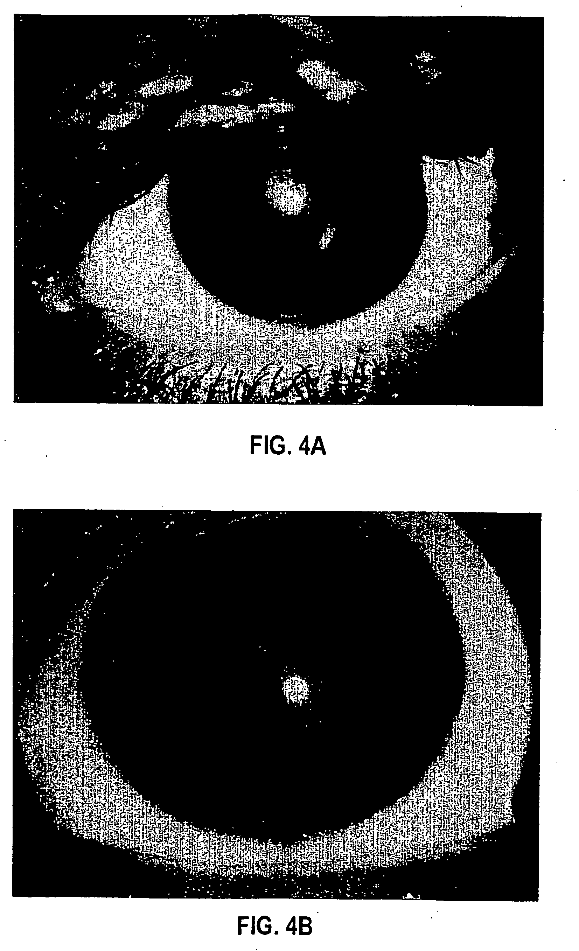

Photographs of anterior segment, FIG. 3A (OD, before treatment), FIG. 3B (OD, after treatment), FIG. 4A (OS, before treatment), and FIG. 4B (OS, after treatment): Observed were an apparent decrease in lens opacity, reduced yellowing of the crystalline lens, and improved corneal clarity.

In addition, the subject stated: "I have used these eye drops for about seven weeks. I can see a golf ball at 300 yards, whereas it was barely visible at 220 yards before. My vision vastly improved, especially in seeing road signs in the distance. I see colors much more brightly and vividly."

### Reference EXAMPLE 7

The ocular pharmacokinetic behavior of EDTA, when administered as a component of Formulation 1, was evaluated in rabbits over a period of five days. Two healthy male rabbits, each approximately 2.5 to 3 kg in body weight, were used for the study.

On day 1 of the study, one drop of Formulation 1 was topically instilled in each eye of both rabbits (four eyes total). No additional eye drops were administered during the course of the study. Samples of aqueous humor were extracted at 15 min, 30 min, 1 hr, 4 hrs, 3 days, and 5 days following administration (as indicated in the following table). Vitreous humor was extracted at 5 days following administration from all four eyes. The concentration of EDTA was measured in all the samples of aqueous humor and vitreous humor by HPLC analysis.

The results of the study are summarized in Table 5.

| Table 5 | | | | |
|---|---|---|---|---|
| | Concentration of EDTA (micrograms per milliliter) | | | |
| | Rabbit 101 | | Rabbit 102 | |
| Aqueous humor: | Right Eye | Left Eye | Right Eye | Left Eye |
| 15 min | 1.3 | | | |
| 30 min | | | 10.7 | |
| 1 hr | | 5.3 | | |
| 4hrs | | | | 0.9 |
| 3 days | 0.5 | 0.4 | 0.5 | 0.7 |
| 5 days | 0.6 | 0.5 | 0.4 | 0.6 |
| Vitreous humor: | | | | |
| 5 days | 0.6 | 0.5 | 0.7 | 0.6 |

Examples 1-7 indicate that topical drops composed of the multifunction agents MSM and EDTA, with the addition of the L-carnosine AGE breakers, significantly improved the quality of both day and night vision (visual acuity), greatly improved contrast sensitivity, improved pupil dilation, produced a more uniform and stable tear film, reduced arcus senilis, and greatly reduced glare and the discomfort associated with photophobia. No adverse pathological changes or reduction in acuity were observed.

### Reference EXAMPLE 8

In the following *in vivo* experiment, the ocular pharmacokinetic behavior of EDTA, when administered with MSM as permeation enhancing penetrating agent, was evaluated in rabbits over a period of five days. Two healthy male rabbits, each approximately 2.5 to 3 Kg in body weight, were used for the study.

An eye drop formulation of the invention, was prepared as follows: High purity de-ionized (DI) water (500 ml) was filtered via a 0.2 micron filter. MSM (27 g), EDTA (13 g), and L-carnosine (5 g) were added to the filtered DI water, and mixed until visual transparency was achieved, indicating dissolution. The mixture was poured into 10 ml bottles each having a dropper cap. On a weight percent basis, the eye drops had the following composition:

| | |
|---|---|
| Purified de-ionized water | 91.74 wt.% |
| MSM | 4.95 wt.% |
| Di-sodium EDTA | 2.39 wt.% |
| L-Carnosine | 0.92 wt.%. |

On day 1 of the study, one drop of Formulation 1 was topically instilled in each eye of both rabbits (four eyes total). No additional eye drops were administered during the course of the study. Samples of aqueous humor were extracted at 15 min, 30 min, 1 hr, 4 hrs, 3 days, and 5 days following administration (as indicated in the following table). Vitreous humor was extracted at 5 days following administration from all four eyes. The concentration of EDTA was measured in all the samples of aqueous humor and vitreous humor by HPLC analysis.

The results of the study are summarized in the following table:

| | Concentration of EDTA (micrograms per milliliter) | | | |
|---|---|---|---|---|
| | Rabbit 101 | | Rabbit 102 | |
| Aqueous humor: | Right Eye | Left Eye | Right Eye | Left Eye |
| 15 min | 1.3 | | | |
| 30 min | | | 10.7 | |
| 1 hr | | 5.3 | | |
| 4 hrs | | | | 0.9 |
| 3 days | 0.5 | 0.4 | 0.5 | 0.7 |
| 5 days | 0.6 | 0.5 | 0.4 | 0.6 |
| Vitreous humor: | | | | |
| 5 days | 0.6 | 0.5 | 0.7 | 0.6 |

These results show that Formulation 1 delivers EDTA to the anterior chamber of the eye (aqueous humor) very rapidly: a concentration of 10.7 µg/mL is reached at only 30 minutes following administration. Because the aqueous humor is completely flushed from the anterior chamber approximately every 90 minutes, compounds from conventional eye drop formulations are typically not detected in the aqueous humor at four hours following administration. We, however, observed significant concentrations of EDTA in the aqueous humor even at five days following administration. Our data also show that EDTA reached the vitreous humor, where it was present in almost the same concentration as in the aqueous humor. It is thus likely that the vitreous humor (and probably adjacent tissues) was acting as a reservoir for the absorbed EDTA, with some of this EDTA diffusing back into the aqueous humor over time.

The demonstrated penetration of EDTA from Formulation 1 into the posterior segment of the eye, including the vitreous humor, indicates the potential of the inventive formulation to deliver therapeutic agents to the posterior of the eye when administered as eye drops. Such drug delivery to the posterior of the eye allows for the treatment of many eye conditions, diseases, and disorders, including age related macular degeneration, macular edema, glaucoma, cell transplant rejection, infections, and uveitis.

### Reference EXAMPLE 9

Formulation 1 was evaluated for efficacy in treating a male subject in his eighties who was suffering from cataracts and SaLzmann's nodules, whose best correction had been 20/400 in his left eye and 20/200 in his right eye, and had acute photophobia and glare, as well as severe macular scarring in the left eye. The formulation was administered to the subject, one drop (approximately 0.04 ml) to each eye, two to four times per day for a period of over 12 months. There were no side effects, other than minor temporary irritation at the time of administering the formulation in the eye, were reported or observed by the subject or the ophthalmologist.

After 4 weeks into the study, the changes reported by the subject included increased brightness, improved clarity of vision, and reduced glare. After 8 weeks the glare and photophobia were reduced, and the subject started to read books, newspapers, and information on the computer screen again. The visual acuity in his right eye improved significantly, from 20/200 (with correction) to 20/60 (pinhole) (with the same correction). In his left eye, his visual acuity improved as well, from 20/400 to 20/200 (with the same correction). In his left eye, he continued to have a central dark spot due to macular scarring.

The subject reported a further reduction in glare and photophobia, and further improvements in the ease of reading books, newspapers, and information on the computer screen. Subject also reported that nighttime glare had been eliminated. The subject was now comfortable in daylight without need for dark glasses, and without suffering severe problems with glare. The visual acuity in his right eye improved from 20/60 (pinhole) to 20/50 (pinhole) In his left eye his visual acuity also improved, from 20/200 to 20/160 (with same correction). In his left eye, he continued to have a central dark spot due to macular scarring.

After eight months, the subject's vision in his right eye improved from 20/50 (pinhole) to 20/40 (pinhole) In his left eye his visual acuity improved from 20/160 to 20/100 (with same correction). The dark spot in the left eye started dissipating, and he could read hazily through the formerly dark spot. At this time his contrast sensitivity was also measured. His cataracts were measured at a 4+ (on a scale of 0-4, 4 being the highest). The central macular scar was barely visible to the ophthalmologist due to haziness of the optical path.

After a total of 12 months, the subject's vision continued to improve. The subject could now read books, newspapers, and the computer screen without any problems. The subject also showed improvement in cataracts (went from 4+ to 3-4+ on a 0-4 scale). The optical path clarity had improved enough that the macular scar was clearly visible to the ophthalmologist. In contrast sensitivity there was a 40% to 100% improvement. In Snellen acuity, from 20/40 to 20/30 (pinhole) in his right eye, and from 20/100 to 20/80 in his left eye. The subject also reported that for the first time in 40 years he could start to see wavy letters through his left eye.

These results demonstrate that the eye-drops are reaching the retina in the back of the eye, and the MSM was aiding the penetration of EDTA and L-Carnosine. These results are consistent with the rabbit study of Reference Example 4.

### Reference EXAMPLE 10

Formulation 1 was evaluated for efficacy in treating a female subject in her sixties who was having problems with "floaters" in both of her eyes. The formulation was administered to the subject, one drop (approximately 0.04 ml) to each eye, two to four times per day for a period of over 12 months. There were no side effects, other than minor temporary irritation at the time of administering the formulation in the eye, were reported or observed by the subject or the ophthalmologist.

After 8 weeks of using the eye drops, the subject reported a significant reduction in the floaters, again confirming that medication was reaching the vitreous, and having a beneficial effect.

### Reference EXAMPLE 11

Formulation **1** was evaluated for efficacy in treating a male subject in his fifties who was had a visual acuity of 20/15 with correction and a very prominent arcus senilis. The formulation was administered to the subject, one drop (approximately 0.04 ml) to each eye, two to four times per day for a period of over 12 months. There were no side effects, other than minor temporary irritation at the time of administering the formulation in the eye, were reported or observed by the subject or the ophthalmologist.

After 16 weeks, the subject reported improvement in visual acuity from 20/25 to 20/15, as well as very significant reduction in his arcus senilis.

### Reference EXAMPLE 12

An eye drop formulation of the invention, Formulation 3, was prepared as follows: Approximately 500 ml of high purity de-ionized (DI) water was filtered via a 0.2 micrometer filter and 27 g of Methylsulfonylmethane (MSM), and 13 g of Ethylenediaminetetraacetic acid disodium salt, dihydrate (EDTA) were added. The formulation was mixed until visual transparency was achieved, the pH was adjusted to 7.2 with NaOH, and the volume was adjusted to 500 ml. The mixture was poured into 10 mL bottles each having a dropper cap. On a weight percent basis, the eye drops had the following composition:

| | |
|---|---|
| Purified de-ionized water | 92.0 wt. % |
| MSM | 5.40 wt. % |
| EDTA disodium salt, dihydrate | 2.60 wt. % |

### Reference EXAMPLE 13

Formulation 3 was evaluated for efficacy for a maximum period of 120 days. Patients were given either Formulation 3 or the placebo (commercially available unpreserved saline) and instructed to use one drop (approximately 0.04 ml) to each eye, four times per day. The patients were randomized to receive either the study formulation or the placebo. Twelve eyes received Formulation 3 while thirteen eyes received the placebo. The study was double-masked, in that neither the patient nor the ophthalmologist knew whether they were given Formulation 3 eye drops or the placebo.

Contrast sensitivity was measured under mesopic conditions simulating dusk (3 candles/m²) using the FACT™ (Functional Acuity Contrast Test) and a CST 1800 Digital® contrast sensitivity tester. Measurements were performed monocularly, in duplicate, for each eye and duplicate measurements were averaged.

The FACT™ uses a sine-wave grating chart to test for contrast sensitivity. The chart consists of five rows (spatial frequencies), each row having nine levels of contrast sensitivity. Sine wave gratings are special test patterns that appear as varying sizes and contrasts of gray bars set up in circular patterns. The gratings in spatial frequency A appear as the largest gray bars (longest wavelength) while the gratings in spatial frequency E appear as the smallest gray bars (shortest wavelength). While viewing the chart through the CST 1800 Digital® contrast sensitivity tester, subjects report the orientation of each grating: right, up or left. For each spatial frequency, there are nine levels of contrast sensitivity, also called patches. Level 1 has the greatest contrast, while level 9 has the least. The subject reports the orientation of the last grating seen (1 through 9) for each row (A, B, C, D and E).

When the FACT is scored, the nine levels of contrast sensitivity are graphed using a logarithmic scale. An improvement of one level or patch represents approximately a 1.5-fold increase in contrast sensitivity. To quantify the contrast sensitivity improvement, data from Day 14 (T₀) were compared to the last contrast sensitivity data obtained for each subject that completed at least 60 days of treatment.

Of the twelve eyes that received Formulation 3, eight eyes (67%) showed a contrast sensitivity improvement of at least two patches in two spatial frequencies, a statistically significant result (p = 0.0237). Of the thirteen eyes that received the placebo, only three (23%) showed an improvement of at least two patches in two spatial frequencies.

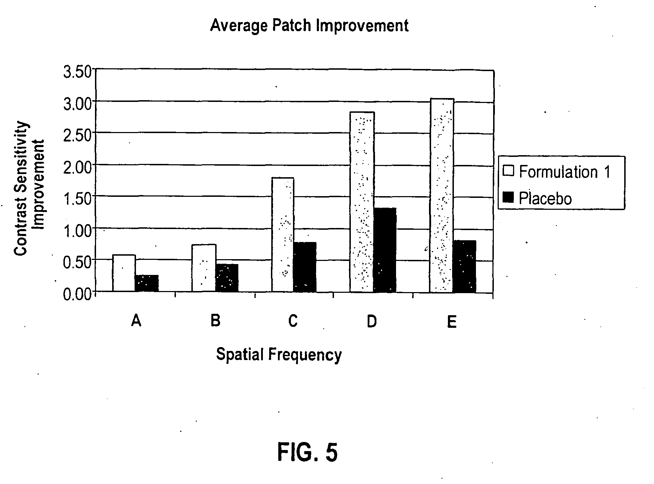

As another measure of contrast sensitivity improvement, the average patch improvement of the eyes that received Formulation 3 was compared to the group of eyes that received the placebo for each spatial frequency (FIG. 5). The eyes that received Formulation 3 showed a significant contrast sensitivity improvement in all spatial frequencies, with an improvement of greater than 2.5 patches in spatial frequency D and an improvement of over 3 patches for spatial frequency E.

None of the subject reported serious ocular or systemic adverse events.

### Reference Example 14

**Objective**. Determine the extend of penetration of ¹⁴C - EDTA into the aqueous of the eye, with and without MSM present, in eye drops applied to rat eyes.

**Reagents.** Ethylenediamine tetraacetic acid-1,2-¹⁴C tetrasodium was purchased from Sigma. ¹⁴C-EDTA (Specific Activity: 10.6 mCi/mmol, radiochemical purity: 99% or higher). All other chemicals used in this study were of analytical grade and purchased commercially. ScintiVerse II Cocktail (Liquid Scintillation Solvent) was general-purpose LSC Cocktail for aqueous, nonaqueous, and emulsion counting systems from Fisher Scientific.

**Animals.** Male Sprague-Dawley rats weighing 200-250 g were obtained from Central Animal Care Services at the University of Texas Medical Branch. The NIH guidelines and ARVO statement for the Use of Animals in Ophthalmic and Vision Research were strictly followed for the welfare of the animals. Rats were sacrificed using 100% carbon dioxide at a low flow rate (25-30% of the volume of the cage per minute) for about 2 minutes.

**Experimental Procedure.** 100 µl of each of the following three eye drop solutions were prepared.
Solution A
80 µl of 5.4% MSM
10 µl of 600 mM EDTA (Tetrasodium salt EDTA)
10 µl of C¹⁴ EDTA (Directly from the bottle)
Solution B:
80 µl of 5.4% MSM
10 µl of 120 mM EDTA (Tetrasodium salt EDTA)
10 µl of C¹⁴ EDTA (Directly from the bottle)
Solution C:
80 µl of PBS
10 µl of 600 mM EDTA (Tetrasodium salt EDTA)
10 µl of C¹⁴ EDTA (Directly from the bottle)

8 µl of each eye drop solution was applied to the cornea of each of the eyes. One rat was treated with each solution. At 0.5, 2, and 16 hours, aqueous humor was aspirated from each eye using a 30-gauge fine needle with an Insulin₋syringe and dispensed in 50 µl of PBS. To solubilize the protein, samples were placed in a 50° C water bath for 3 hours followed by centrifugation at 10,000 rpm for 10 minutes.

**Determination of the radioactivity of the samples**. Samples were added to the counting vials containing 25 ml of ScintiVerse II counting fluid, mixed vigorously and allowed to stand for 1 hour in the dark. The samples were then counted using a Liquid Scintillation counter (LS 1801 Liquid Scintillation Systems, Beckman Instruments, Inc.). Counts per minute were averaged for the two eyes that received each solution for each time point.

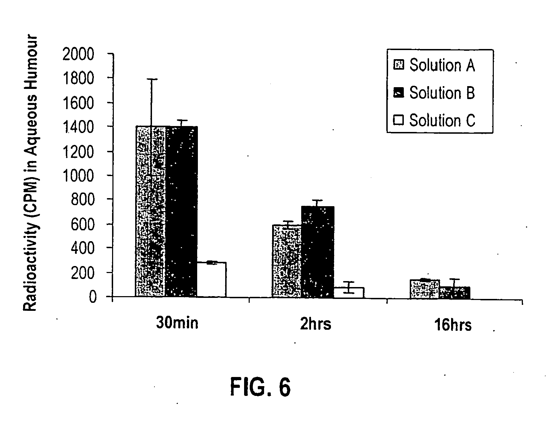

To evaluate the ability of each solution to be transported from the cornea to the aqueous humor, the amount of ¹⁴C-EDTA in the aqueous humor was compared between Solutions A, B, and C (FIG. 6). In the absence of MSM, very little EDTA was present in the aqueous humor, regardless of the EDTA concentration. At the 30-minute time point, there was an increase of approximately 5-fold in the amount of ¹⁴C-EDTA in the aqueous humor in the presence of MSM.

### Reference EXAMPLE 15

### EDTA PHARMACOKINETIC STUDY

**Objective**. Determine the amount of C-14 labeled EDTA that penetrates into the various structures of the rat eye (cornea, aqueous humor, lens, vitreous, and retina), using eye drops that contain MSM. A comparison of two different eye drop formulations that differed in their EDTA concentration.

**Reagents**. Ethylenediaminetetra acetic acid-1,2-¹⁴C tetrasodium was purchased from Sigma. ¹⁴C-EDTA (Specific Activity: 10.6 mCi/mmol, radiochemical purity: 99% or higher). All other chemicals used in this study were of analytical grade and purchased commercially. ScintiVerse II Cocktail (Liquid Scintillation Solvent) was general-purpose LSC Cocktail for aqueous, nonaqueous and emulsion counting systems from Fisher Scientific.

**Animals.** Male Sprague-Dawley rats weighing 200-250 g were obtained from Central Animal Care Services at the University of Texas Medical Branch. The NIH guidelines and ARVO statement for the Use of Animals in Ophthalmic and Vision Research were strictly followed for the welfare of the animals. Rats were sacrificed using 100% carbon dioxide at a low flow rate (25-30% of the volume of the cage per minute) for about 2 minutes.

**Eye Drop 1.**
60.5mM EDTA
10µl, 5 µCi of ¹⁴C-EDTA;
10 µl of 600mM EDTA;
80 ul of 5.4% MSM.

**Eye Drop 2**.
12.5 mM EDTA
10 µl, 5 µCi of ¹⁴C-EDTA;
10 µl of 120 mM EDTA;
80 µl of 5.4% MSM.

8 µl of Eye Drop 1 was applied to the rats' eyes. After 0.5, 1, 2, 4, and 16 hours, the rats were sacrificed and the eyeballs removed. The eyeballs were quickly washed 6 times in 5 ml of saline each time. Aqueous humor was aspirated from both eyes and dispensed in 50 µl of PBS. Cornea, lens, vitreous and retina from each eye were separated and placed in Eppendorf tubes containing H₂O and 10N NaOH in the following ratio:

| | |
|---|---|
| Cornea: | 200 µl H₂O + 40 µl of 10N NaOH; |
| Lens: | 500 µl H₂O + 100 µl of 10N NaOH; |
| Vitreous: | 200 µl H₂O + 40 µl of 10N NaOH; |
| Retina: | 200 µl H₂O + 40 µl of 10N NaOH. |

To solubilize the protein, samples were placed in a 50°C water bath for 3 hours followed by centrifugation at 10,000 rpm for 10 minutes. Samples were added to the counting vials containing 25ml of ScintiVerse II counting fluid, mixed vigorously and allowed to stand for 1 hour in the dark. They were then counted using a Beckman Scintillation counter (LS 1801 Liquid Scintillation Systems, Beckman Instruments, Inc.).

8 µl of Eye Drop 2 was applied to the rat eye. After 0.5, 2 and 4 hours, the rats were sacrificed and the experiment conducted in the same way as for Eye Drop 1.

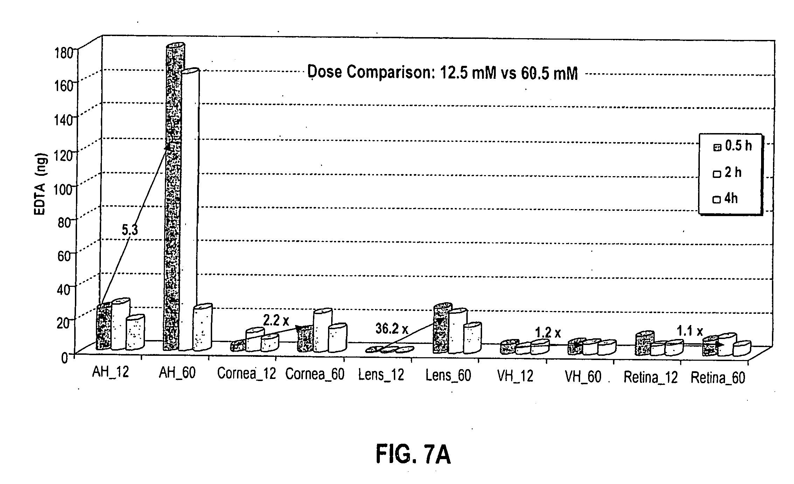

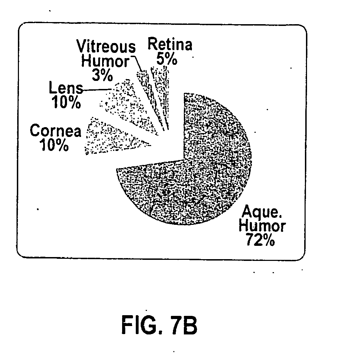

To look at the distribution of each formulation in the eye structures, the number of nanograms of EDTA was calculated for each time point (FIG. 7A). Dose dependency was observed, particularly in the aqueous humor, the cornea, and the lens. The percentage of EDTA found in each eye structure was calculated for the two-hour time point for Eye Drop 1 (FIG. 7B). The majority of the EDTA was found in the aqueous humor, however, the Eye Drop 1 formulation was present in all tissues examined.

### Reference EXAMPLE 16

### EVALUATION OF OXIDATION-INDUCED TOXICITY IN RAT LENS ORGAN CULTURE (RLCE)

Materials. EDTA, Ascorbic acid, and H₂O₂ were purchased from Sigma. All cell culture medium components were from Invitrogen.

Animal. Male Sprague-Dawley rats weighing 200-250 g were obtained from Central Animal Care Services at the University of Texas Medical Branch. The NIH guidelines and ARVO statement for the Use of Animals in Ophthalmic and Vision Research were strictly followed for the welfare of the animals. Rats were sacrificed with using 100% carbon dioxide at a low flow rate (25-30% of the volume of the cage per minute) for about 2 minutes.

**Lens Culture.** The rat lenses were dissected and washed with 1% penicillin/streptomycin in sterile PBS. The lenses were cultured in medium 199 containing 0.1% gentamicin at 37°C in a 5% CO₂ humidified atmosphere. The lenses were divided into groups of two lenses each and were exposed to either glucose or ascorbate with H₂O₂ MSM and/or EDTA. The medium was changed every day for 7 days. The lenses were visualized under a Nikon Eclipse 200 and photographs were taken using a Multidimensional Imaging System.

**Table 6 - Preparation of Reagents.**

| | |
|---|---|
| Medium M199 + 0.1% gentamicin | 250 ml of M199 + 250 µl of gentamicin |
| 400 mM MSM (FW 94.2) | 376 mg MSM + PBS to final volume to 10 ml |
| 50 mM EDTA (Tetrasodium Salt FW 380) | 190 mg EDTA + PBS 8 ml, adjust pH to 7.2 with HCl, adjust final volume to 10 ml. |
| 2.5 M glucose (FW 180) | 900 mg glucose + 2 ml ddH₂O |
| 100 mM ascorbate (FW174) | 176 mg ascorbate + 10 ml ddH₂O |
| 10 mm H₂O₂ | 11 µl of 30% H₂O₂ + ddH₂O to final volume 10 ml. |

**Experimental Procedure.** 1. Sacrificed seven rats, removed the eyeballs as soon as possible, and put them into a tube containing PBS with 0.1% gentamicin. 2. Dissected the lenses immediately and washed with PBS. 3. Transferred all lenses to two 12-well plates (2 ml of medium per well/per lens). Each treatment was performed in 2 wells. Final concentrations for the six treatments were as follows:
50 mM glucose
50 mM glucose + 4 mM MSM
50 mM glucose + 4 mM MSM + 0.5 mM EDTA
1 mM ascorbate + 100 µM hydrogen peroxide
1 mM ascorbate + 100 µM hydrogen peroxide + 4 mM MSM
1 mM ascorbate + 100 µM hydrogen peroxide + 4 mM MSM + 0.5 mM EDTA 4. The medium and the reagents were changed every day. 5. After 7 days of lens culture, took photographs and determined level of light transparency through the lenses.

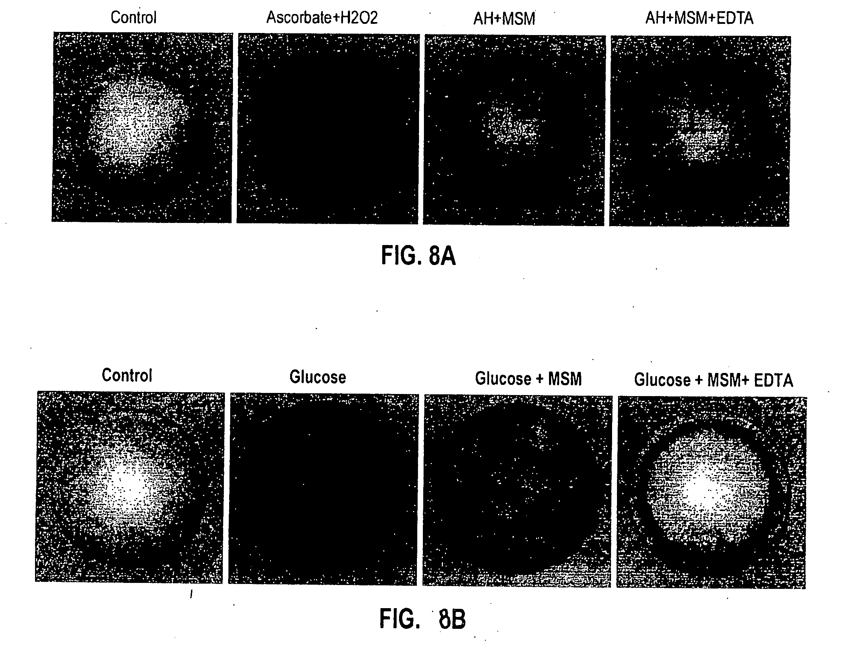

**Results**. Photographs of the lens culture showed that significant rat lens opacity was induced with both glucose and ascorbate plus hydrogen peroxide (FIGS. 8A and 8B). MSM mitigated lens opacification by both oxidants; however, MSM plus EDTA provided the most effective protection.

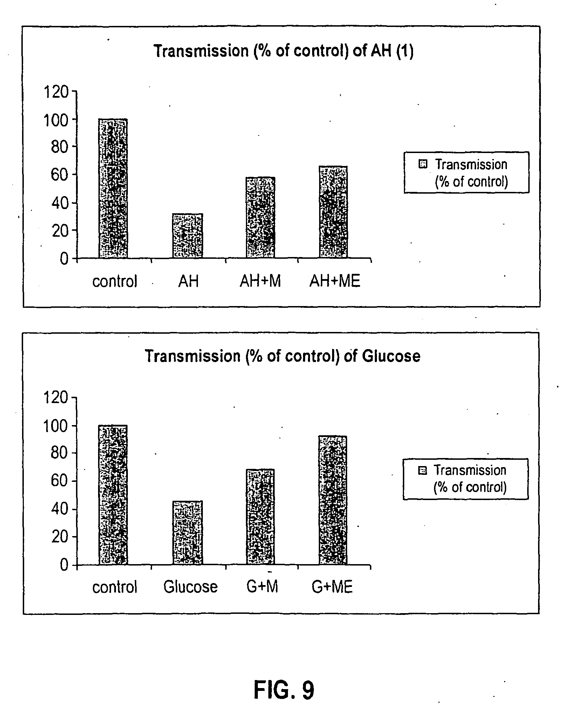

The level of light transmission through the lens was used to quantify lens opacity for each treatment. Consistent with the photographic results, MSM improved the level of light transmission for both oxidative treatments, while MSM + EDTA gave an even greater improvement (FIG. 9). Light transmission through the lens treated with ascorbate/hydrogen peroxide (AH) was 32% of light transmission through the control (upper graph). Light transmission through the lenses treated with ascorbate/hydrogen peroxide and MSM (AH + M) and ascorbate/hydrogen peroxide and MSM/EDTA (AH + ME) were 57% and 66% respectively. A similar pattern was observed when 50 mM Glucose was used as the oxidant (lower graph). Light transmission through the lens treated with glucose was only 45% of light transmission through the untreated control. Light transmission through the lenses treated with glucose plus MSM (G + M) and glucose and MSM/EDTA (G + ME) were 68% and 92% respectively.

### Reference EXAMPLE 17

### EVALUATION OF CELL VIABILITY FOLLOWING OXIDATION-INDUCED TOXICITY IN HUMAN LENS EPITHELIAL CELLS (HLEC) AND PROTECTION WITH MSM AND/OR EDTA

**Materials**. EDTA (Tetrasodium Salt), Ferrous ammonium sulfate, Ferric chloride, Adenosine 5'-diphosphate (ADP), Ascorbic acid, and H₂O₂ were purchased from Sigma. All cell culture medium components were from Invitrogen.

**Cell Culture and Treatment.** Human lens epithelial cells (HLECs) with extended life span were cultured in DMEM medium containing 0.1% Gentamicin and supplemented with 20% fetal bovine serum at 37°C in a 5% CO₂ humidified atmosphere. 1.0×10⁵ HLECs /ml (Passage 5) were seeded in 12-well plate overnight prior to the addition of oxidation reagents and MSM and/or EDTA.

**Cell Viability**. Cell survival was determined by Trypan Blue staining and counting with a hemocytometer. Dead cells stain blue, while live cells exclude Trypan Blue. Cell viability is represented as percentage of the number of live cells/number of total cells.

**Preparation of Reagents.**

| | |
|---|---|
| HLEC medium | DMEM + 20% FBS + 0.1% gentamicin |
| 400 mM MSM | 376 mg/10 ml PBS for stock |
| 50 mM EDTA (Tetrasodium Salt) | 190 mg/10 ml PBS for stock, pH 7.2 |
| Hydrogen peroxide | 30 mM stock |
| 5 M Glucose | 1800 mg/10 ml of ddH₂O |
| 100 mM Ascorbate | 176 mg/10 ml of ddH₂O |
| Fenton | Ferrous ammonium sulfate (FAS) 1 mM, ADP 10 mM, H₂O₂ 10 mM |
| Fenton' | FAS 1 mM, ADP 10 mM, H₂O₂ 10 mM |
| Ferric Chloride | FeCl₃ 5mM, EDTA 5mM, H₂O₂ 20mM |

**Experimental Procedure.** 1. Seeded 0.5×10⁵ /ml of HLEC (Passage 5) into three 12-well plates, incubated at 37°C for overnight. 2. Changed medium to 2% FBS DMEM medium. 3. Added the oxidation reagents and MSM and/or EDTA to the proper wells. Final concentrations were as follows:
4 mM MSM
0.5 mM EDTA
100 µM H₂O₂
50 mM glucose
1 mM ascorbate
Fenton: Ferrous ammonium sulfate (FAS) 10 µM, ADP 100 µM, H₂O₂ 100 µM
Fenton': FAS 10µM, ADP 100 µM, H₂O₂ 100 µM
Ferric Chloride: FeCl₃ 50 µM, EDTA 50 µM, H₂O₂ 200 µM

After adding oxidation reagents and MSM and/or EDTA, cells were incubated at 37°C with 5% CO₂ and 95% air for 16 hrs, and harvested with 0.25%Trypsin-EDTA and cell viability determined with Trypan-Blue.

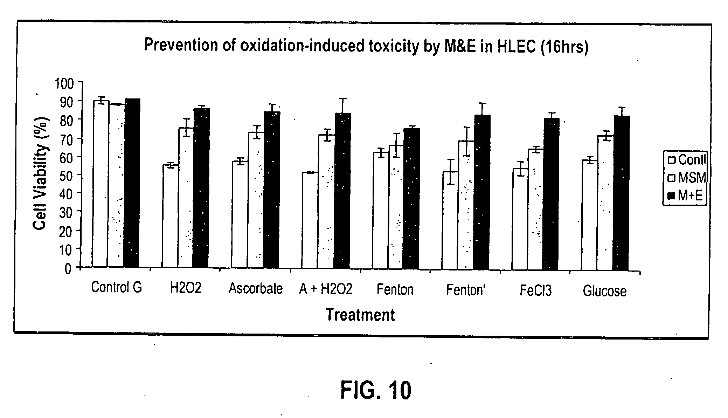

**Results**. FIG. 10 shows the percent of cell viability under each condition. The oxidants decreased cell viability between 30% (Fenton) and almost 45% (ascorbate + H₂O₂). The addition of 4 mM MSM increased the percent cell viability for all oxidants, while the addition of 4 mM MSM with 0.5 mM EDTA gave a greater increase in the percentage of viable cells. A Chi Square test was performed to determine whether the protective effects of MSM/EDTA were statistically significant For those wells containing an oxidant plus the MSM/EDTA mixture, statistically significant results (P value of less than 0.05) were obtained for all oxidants except Fenton.

### Reference EXAMPLE 18

### INTESTINAL MEMBRANE PERMEATION WITH MSM AS PERMEATION ENHANCER AND METHYLENE BLUE AS ACTIVE

The following example was designed to show how the presence of MSM in the donor solution of an *in vitro* drug permeation experiment improves the permeation of other formula ingredients (actives) through animal membrane tissues. In this experiment we selected methylene blue as the species for which permeation enhancement was desired. As tissue model for this experiment we selected porcine intestinal membrane.

A small glass vial was filled with test solution and capped with the test membrane (tissue layer of defined thickness). The membrane tissue was hold in place with a rubber O-ring and a screw cap of defined surface area opening. This test unit was immersed into a large glass vial filled with receptor medium. From this large glass vial, small sample receptor medium volumes were periodically removed for analysis.

**Table 6** shows the experimental parameters for Example 18.

**Table 6: Experimental parameters for Example 18.**

| **Materials** | | |
|---|---|---|
| Membrane | Porcine Intestinal Membrane Casing - used in sausage MFG | |
| Active | methylene blue, 0.5 mg/mL water | |
| Plus Enhancer | MSM, 27 mg/mL water | |

| Experimental | | |
|---|---|---|
| Donor vial | 2 ml screw cap HPLC vials with septum cap | |
| Gasket | Rubber O-rings to secure membrane | |
| Receptor vials | 100 ml water, Periodically remove samples for analysis | |
| Analysis | Bausch & Lomb Spectronic 21, Absorbance at 668 nm | |
| Control | methylene blue, 0.5 mg/mL water | |

**Table 7** shows the analytical data for Reference Example 18.

**Table 7: Analytical data for Reference Example 18**

| **Time (Hr)** | **Control** | **MSM** |
|---|---|---|
| 0.55 | 0.021 | 0.023 |
| 1.9 | 0.061 | 0.176 |
| 2.9 | 0.162 | 0.323 |
| 4 | 0.234 | 0.433 |
| 5.7 | 0.283 | 0.563 |
| 6.9 | 0.408 | 0.738 |

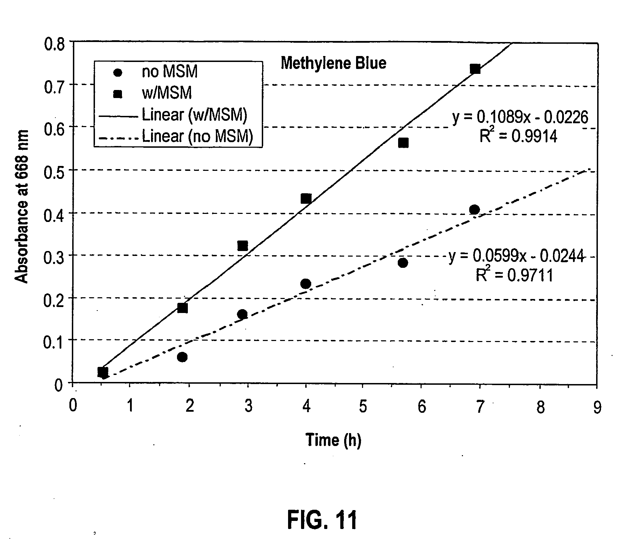

As shown in FIG. 11, the cumulative permeation of methylene blue through porcine intestinal membrane is enhanced by approximately a factor of 2 when MSM is present in the donor solution. The drug diffusion follows a linear relationship, as predicted by Fick's law for passive diffusion.

As proven by this experiment, the presence of MSM is capable of increasing the permeation rate of methylene blue through porcine intestinal membrane.

### Reference EXAMPLE 19

### BOVINE CORNEA PENETRATION WITH MSM AS PERMEATION ENHANCER AND ETHYLENE BLUE AS ACTIVE

In this example the same principal study design and protocol was used as described in Example 18. In this experiment, we selected methylene blue again as the species for which permeation enhancement was desired; however, as tissue model we selected bovine cornea. The bovine cornea was dissected from bovine eyes procured from slaughterhouses. We selected bovine cornea tissue as the test membrane because it is known to be a very difficult membrane to penetrate. Bovine cornea is about 4-6 times thicker than the human corneal membrane.

**Table 8** shows the experimental parameters for Reference Example 19 Example 19.

**Table 8: Experimental parameters for Reference Example 19**

| **Materials** | | |
|---|---|---|
| Membrane | Bovine Cornea dissected from bovine eye | |
| Active | methylene blue, 0.5 mg/mL water | |
| Plus Enhancer | MSM, 27 mg/mL water | |

| **Experimental** | | |
|---|---|---|
| Donor vial | 2 ml screw cap HPLC vials with septum cap | |
| Gasket | Rubber O-rings to secure membrane | |
| Receptor vials | 100 ml water, Periodically remove samples for analysis | |
| Analysis | Bausch & Lomb Spectronic 21, Absorbance @ 668 nm | |
| Control | methylene blue, 0.5 mg/mL water | |

**Table 9** shows the analytical data for Example Reference 19.

**Table 9: Analytical data for Reference Example 19**

| **Time (Hr)** | **Control** | **MSM** |
|---|---|---|
| 43.5 | 0.024 | 0.093 |
| 52.75 | 0.03 | 0.13 |
| 65 | 0.035 | 0.163 |
| 75.5 | 0.042 | 0.195 |
| 89 | 0.057 | 0.213 |
| 97.5 | 0.076 | 0.265 |

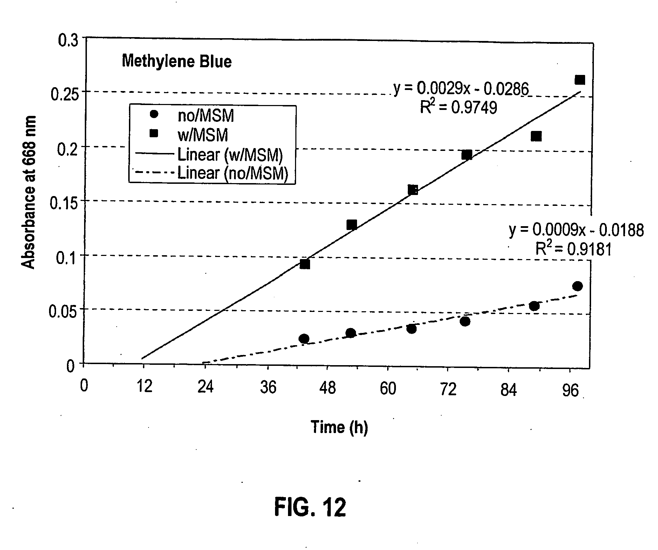

As shown in FIG. 12, the cumulative permeation of methylene blue through bovine cornea is enhanced by more than a factor of 3 when MSM is present in the donor solution. The drug diffusion follows a linear relationship, as predicted by Fick's law for passive diffusion. Furthermore, the lag time is reduced from about 24 hours to less than 12 hours.

### EXAMPLE 20

### INTESTINAL MEMBRANE PERMEATION WITH MSM AS PERMEATION ENHANCER AND CIPROFLOXACIN AS ACTIVE

In this example the same principal study design and protocol was used as described in Example 18. Here we selected the antibiotic ciprofloxacin as the species for which permeation enhancement was desired. The tissue model was porcine intestinal membrane.

**Table 10** shows the experimental parameters for Example 20.

**Table 10: Experimental parameters for Example 20**

| **Materials** | | |
|---|---|---|
| Membrane | Porcine Intestinal Membrane Casing used in sausage manufacturing | |
| Active | Ciprofloxacin, 0.5 mg/mL water | |
| Plus Enhancer | MSM, 90 mg/mL water | |

| **Experimental** | | |
|---|---|---|
| Donor vial | 2 ml screw cap HPLC vials with septum cap | |
| Gasket | Rubber O-rings to secure membrane | |
| Receptor vials | 100 ml water, Periodically remove samples for analysis | |
| Analysis | Bausch & Lomb Spectronic 21, Absorbance @ 275 nm | |
| Control | Ciprofloxacin, 0.5 mg/mL water | |

**Table 11** shows the analytical data for Example 20.

**Table 11: Analytical data for Example 10**

| **Time (Hr)** | **Control** | **MSM** |
|---|---|---|
| 0.17 | 0.041 | 0.039 |
| 0.37 | 0.046 | 0.066 |
| 0.60 | 0.055 | 0.092 |
| 0.83 | 0.065 | 0.112 |
| 1.17 | 0.089 | 0.163 |
| 1.37 | 0.099 | 0.18 |
| 1.67 | 0.101 | 0.191 |
| 1.85 | 0.115 | 0.224 |
| 2.00 | 0.118 | 0.255 |
| 3.83 | 0.185 | 0.388 |

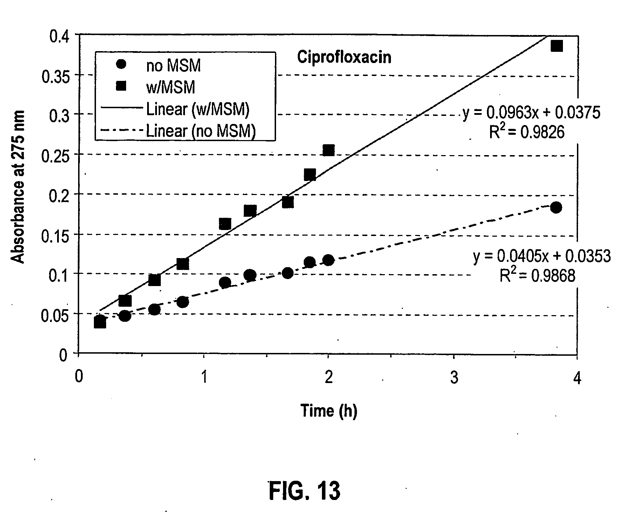

As shown in FIG. 13, the cumulative permeation of ciprofloxacin through porcine intestinal membrane is enhanced by more than a factor of 2 when MSM is present in the donor solution. The drug diffusion follows a linear relationship, as predicted by Fick's law for passive diffusion.

As proven by this experiment the presence of MSM is capable of increasing the permeation rate of ciprofloxacin through porcine intestinal membrane.

### EXAMPLE 21

### INTESTINAL MEMBRANE PERMEATION WITH MSM AS PERMEATION ENHANCE AND CIPROFLOXACIN-HCL AS ACTIVE AND EDTA AS ADDITIONAL INGREDIENT

In this example the same principal study design and protocol was used as described in Example 18. Here we selected the antibiotic ciprofloxacin-HCl as the species for which permeation enhancement was desired. An additional ingredient (EDTA) was added to the solution to explore its effect. The tissue model was porcine intestinal membrane.

**Table 12** shows the experimental parameters for Example 21.

**Table 12: Experimental parameters for Example 21**

| **Materials** | | |
|---|---|---|
| Membrane | Porcine Intestinal Membrane Casing - used in sausage MFG | |
| Active | Ciprofloxacin-HCl, 0.54 mg/mL water | |
| Plus Enhancer | MSM, 150 mg/mL water | |
| Plus other | EDTA, 26 mg EDTA/ ml water | |

| **Experimental** | | |
|---|---|---|
| Donor vial | 2 ml screw cap HPLC vials with septum cap | |
| Gasket | Rubber O-rings to secure membrane | |
| Receptor vials | 100 ml water, Periodically remove samples for analysis | |
| Analysis | Bausch & Lomb Spectronic 21, Absorbance @ 275 nm | |
| Control A | Ciprofloxacin-HCl no MSM, no EDTA | |
| Control B | Ciprofloxacin-HCl no MSM, w/EDTA | |
| Control C | Ciprofloxacin-HCl w/MSM, no EDTA | |

**Table 13** shows the analytical data for Example 21.

**Table 13: Analytical data for Example 21**

| Time | A: no MSM/EDTA | B: no MSM w/EDTA | C: w/MSM no EDTA | w/MSM+EDTA |
|---|---|---|---|---|
| 0.1 | 0.031 | 0.039 | 0.037 | 0.035 |
| 0.4 | 0.05 | 0.058 | 0.059 | 0.077 |
| 0.8 | 0.102 | 0.137 | 0.134 | 0.126 |
| 1.4 | 0.123 | 0.204 | 0.196 | 0.177 |
| 2 | 0.162 | 0.273 | 0.25 | 0.239 |
| 3.1 | 0.218 | 0.402 | 0.41 | 0.362 |
| 5. | 0.335 | 0.559 | 0.579 | 0.515 |

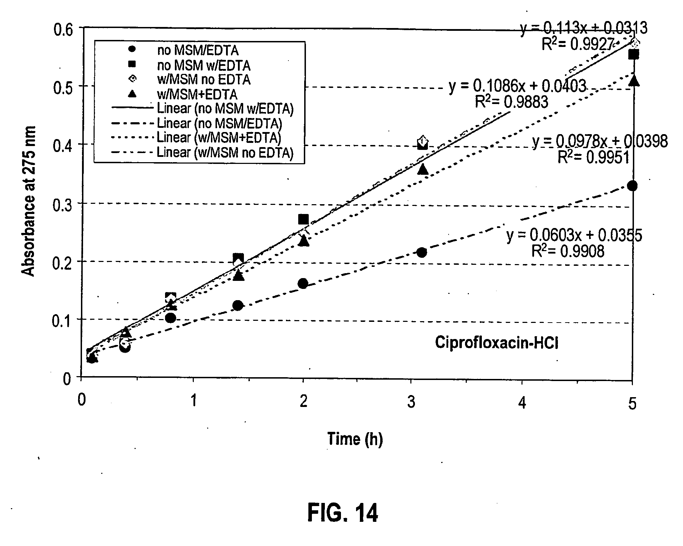

As shown in FIG. 14, the cumulative permeation of ciproffoxacin-HCl through porcine intestinal membrane is enhanced by about a factor of two when MSM or EDTA or both are present in the donor solution. The drug diffusion follows a linear relationship, as predicted by Fick's law for passive diffusion.

As proven by this experiment, the presence of MSM is capable of increasing the permeation rate of ciprofloxacin-HCl through porcine intestinal membrane. EDTA has an enhancing effect, too. However, when both compounds are present in the donor solution, the permeation rate of ciprofloxacin-HCl is not further increased beyond that of the single enhancer solution.

### EXAMPLE 22

### INTESTINAL MEMBRANE PERMEATION WITH MSM AS PERMEATION ENHANCER AND CIPROFLOXACIN-HCL AS ACTIVE AND EDTA AS ADDITIONAL INGREDIENT (REPEAT OF EXAMPLE 21)

This is a direct repeat of Example 21, with the membrane was replaced by a different lot.

**Table 14** shows the experimental parameters for Example 22.

**Table 14: Experimental parameters for Example 22**

| **Materials** | | |
|---|---|---|
| Membrane | Porcine Intestinal Membrane Casing - Lot#2 | |
| Active | Ciprofloxacin-HCl, 0.54 mg/mL water | |
| Plus Enhancer | MSM, 90 mg/mL water | |
| Plus other | EDTA, 26 mg EDTA/ ml water | |

| **Experimental** | | |
|---|---|---|
| Donor vial | 2 ml screw cap HPLC vials with septum cap | |
| Gasket | Rubber O-rings to secure membrane | |
| Receptor vials | 100 ml water, Periodically remove samples for analysis | |
| Analysis | Bausch & Lomb Spectronic 21, Absorbance @ 275 nm | |
| Control A | Ciprofloxacin-HCl no MSM, no EDTA | |
| Control B | Ciprofloxacin-HCl no MSM, w/EDTA | |
| Control C | Ciprofloxacin-HCl w/MSM, no EDTA | |

**Table 15** shows the analytical data for Example 22.

**Table 15: Analytical data for Example 22**

| **Time** | **A: no MSM/EDTA** | **B: no MSM w/EDTA** | **C: w/MSM no EDTA** | **w/MSM+EDTA** |
|---|---|---|---|---|
| 0.1 | 0.031 | 0.039 | 0.037 | 0.035 |
| 0.4 | 0.05 | 0.058 | 0.059 | 0.077 |
| 0.8 | 0.102 | 0.137 | 0.134 | 0.126 |
| 1.4 | 0.123 | 0.204 | 0.196 | 0.177 |
| 2 | 0.162 | 0.273 | 0.25 | 0.239 |
| 3.1 | 0.218 | 0.402 | 0.41 | 0.362 |
| 5 | 0.335 | 0.559 | 0.579 | 0.515 |
| 0.2 | 0.043 | 0.051 | 0.056 | 0.064 |
| 0.7 | 0.066 | 0.09 | 0.104 | 0.107 |
| 1.5 | 0.13 | 0.169 | 0.203 | 0.2 |
| 3 | 0.217 | 0.299 | 0.369 | 0.34 |
| 4.3 | 0.272 | 0.412 | 0.514 | 0.46 |

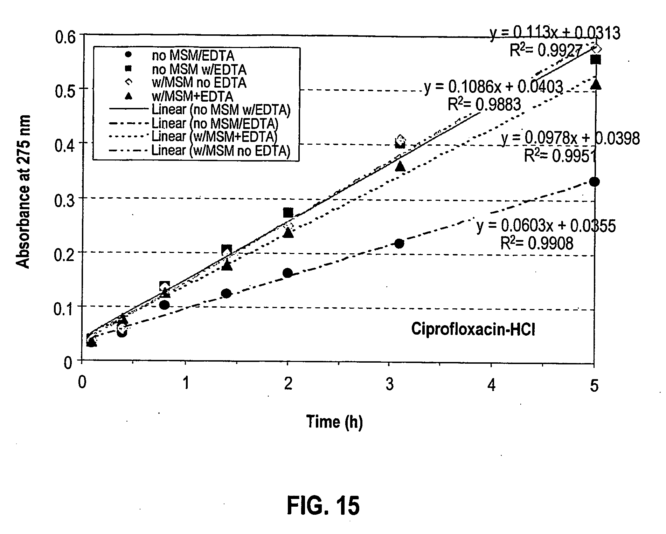

As shown in FIG. 15, the cumulative permeation of ciproffoxacin-HCl through porcine intestinal membrane is enhanced by about a factor of two when MSM or EDTA or both are present in the donor solution. The drug diffusion follows a linear relationship, as predicted by Fick's law for passive diffusion.

As proven by this experiment, the presence of MSM is capable of increasing the permeation rate of ciprofloxacin-HCl through porcine intestinal membrane. EDTA has an enhancing effect, too. However, when both compounds are present in the donor solution, the permeation rate of ciprofloxacin-HCl is not further increased beyond that of the single enhancer solution. The experiment shows excellent reproducibility.

### Reference EXAMPLE 23

### INTESTINAL MEMBRANE PERMEATION WITH MSM AS PERMEATION ENHANCER AND L-CARNOSINE AS ACTIVE AGENT

The same study design and experimental setup was used as described in the above experiments.

At 27 mg MSM/ml, there was no effect on the diffusion rate of L-carnosine. Only at a high MSM concentration (90 mg MSM/mL) was there a measurable modest increase of permeation (about 2.5% over control).

### Reference EXAMPLE 24

### INTESTlNAL MEMBRANE PERMEATION WITH MSM AS PERMEATION ENHANCER AND GLUTATHIONE AS ACTIVE

The same study design and experimental setup was used as described in the above experiments.

At 54 mg MSM/ml, there was no effect on the diffusion rate of glutathione.

### Reference EXAMPLE 25

### INTESTINAL MEMBRANE PERMEATION WITH MSM AS PERMEATION ENHANCER AND DEXAMETHASONE AS ACTIVE

The same study design and experimental setup was used as described in the above experiments.

At 27 mg MSM/ml, there was a 25% increase on the diffusion rate of dexamethasone. At high MSM concentrations (200 mg MSM/mL), the dexamethasone permeation increased by approximately 50% over control.

### Reference EXAMPLE 26

### DEXAMETHASONE SOLUBILITY EXPERIMENT

The water solubility of dexamethasone at room temperature is 10 mg/100 mL. At an MSM concentration of 30 mg/mL, the dexamethasone is not significantly increased. Increasing the MSM concentration to 250 mg/mL, dexamethasone solubility increased to 45 mg/100 mL.

Conclusion: MSM at higher concentrations had a significant effect on dexamethasone solubility.

**Table 16: Solubility of Dexamethasone with Increasing MSM Concentration**

| **MSM (mg/ml)** | **Dexamethasone (mg/ml)** |
|---|---|
| 30 | 0.16 |
| 50 | 0.175 |
| 100 | 0.19 |
| 150 | 0.27 |
| 200 | 0.34 |
| 250 | 0.45 |

### Reference EXAMPLE 27

### MSM PERMEATION ENHANCEMENT MODALM ASSESSMENT - SOLVENT-TYPE ENHANCER

This experiment was designed to investigate if there is a long-lasting enhancement effect of MSM, i.e., if there is a long-lasting disruption of membrane permeability after the formula was removed or washed-out after application.

There are two major classes of permeation enhancers: plasticizer-type enhancers that disrupt the order or structure of the diffusion barrier to create "spaces" for the active to permeate, and solvent-type enhancers that increase the solubility profile in the tissue of interest.

MSM is a small molecule, with a structure somewhat analogous to water (small molecule with oxygen available for hydrogen bonding with water and other molecules) as well as having 2 methyl groups, which can interact with hydrocarbons of organic molecules. MSM does not have large hydrocarbon chains which can disrupt membranes, so it is not likely to act as a "plasticizer-type" enhancer. The most likely mode of action for MSM is that it acts like a "solvent-type" enhancer. Among solvents, MSM is unusual because it is a solid, it is very soluble in water, it can easily form hydrogen bonds with other molecules, including water (based on its structure), and it has a degree of hydrophobicity due to its two methyl groups. The experiment with dexamethasone demonstrated its "solvent" ability. Being a solvent-type enhancer and being so soluble, MSM's residence time in the tissue is thought to be limited and only existing if a donor solution is present.

In this example, the same principal study design and protocol was used as described in Example 8. Methylene blue was the active. The tissue model was porcine intestinal membrane, which was pre-treated. In this experiment all membranes were soaked for 24 hours in a MSM containing pre-treatment solution. Only for the "Control" and "MSM-Enhanced" experiment, the soaking solution did not contain MSM. After the 24-hour soaking period, "Test A" membrane was thoroughly washed, while "Test B" membrane was only briefly rinsed. Those membrane specimens were then mounted as described earlier on methylene blue containing glass vials, which were then immersed as described earlier into a larger glass vial containing receptor medium containing. Only the MSM-enhanced donor solution contained, besides methylene blue, also MSM for enhancement purposes.

| | |
|---|---|
| Control: | No MSM in 24-hour pre-treatment, no MSM enhancer in donor |
| Test A: | 24-hour MSM pretreatment w/thorough washing, no MSM enhancer |
| Test B: | 24-hour MSM pretreatment w/brief rinsing, no MSM enhancer |
| MSM-enhanced: | No MSM in 24-hour pre-treatment, w/MSM enhancer |

**Table 17** shows the experimental parameters for the current example:

**Table 17: Experimental parameters for Example 27**

| **Materials** | | |
|---|---|---|
| Membrane | Porcine Intestinal Membrane Casing - used in sausage manufacturing | |
| Active | Methylene blue, 0.5 mg/mL water | |
| Plus Enhancer | MSM, 50 mg/mL water | |
| Soaking Medium | No MSM in 24-hour pre-treatment | |

| **Experimental** | | |
|---|---|---|
| Donor vial | 2 ml screw cap HPLC vials with septum cap | |
| Gasket | Rubber O-rings to secure membrane | |
| Soaking Medium | MSM, 50 mg/mL water | |
| Pre-treatment | 24 hours | |
| Receptor vials | 100 ml water, Periodically remove samples for analysis | |
| Analysis | Bausch & Lomb Spectronic 21, Absorbance @ 668 nm | |
| Control | No MSM pre-treatment, no MSM enhancer | |
| Test A | MSM pretreatment w/thorough washing, no MSM enhancer | |
| Test B | MSM pretreatment w/brief rinsing, no MSM enhancer | |

**Table 18** shows the analytical data for the present example.

**Table 18: Analytical data for Reference Example 27**

| **Time** | **Control** | **Test A** | **Test B** | **MSM-enhanced** |
|---|---|---|---|---|
| 0.27 | 0.01 | 0.01 | 0.019 | |
| 0.50 | 0.025 | 0.031 | 0.041 | 0.06 |
| 0.75 | 0.037 | 0.043 | 0.055 | |
| 1.00 | 0.052 | 0.064 | 0.079 | 0.13 |
| 1.50 | 0.078 | 0.089 | 0.118 | 0.204 |
| 2.00 | 0.114 | 0.125 | 0.16 | 0.281 |
| 24.00 | 0.92 | 0.939 | 1.112 | 1.393 |

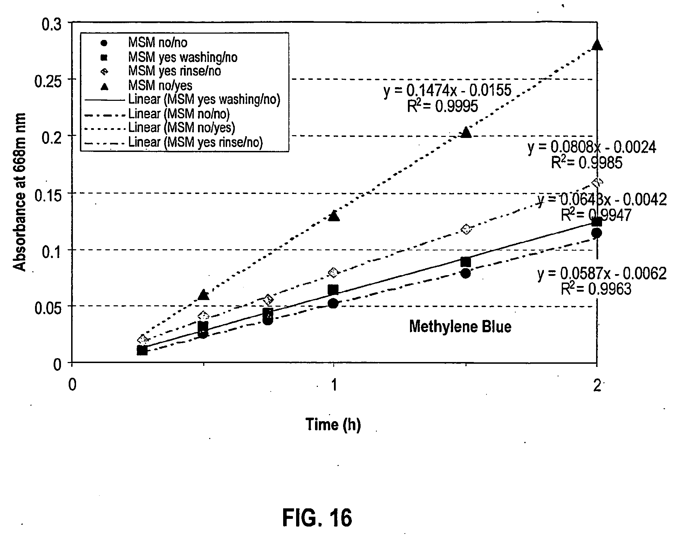

In FIG. 16, the cumulative permeation of methylene blue though pre-treated porcine intestinal membrane is shown. As observed before, the enhanced solution generates an approximately 2 times larger methylene blue permeation rate. The MSM-pretreated membrane sample shows a slightly higher methylene permeation, as one would predict due to the still remaining MSM in the tissue. When the tissue was washed thoroughly, there was no detectable remaining enhancement effect and the observed permeation rate was essentially the same as for virgin tissue, although some remaining enhancement can be discerned.

As proven by this experiment, the presence of MSM is capable of increasing the permeation rate of methylene blue through porcine intestinal membrane. There is some remaining enhancement when the membrane was pre-loaded with MSM and no thorough washing (elution of the MSM from the membrane tissue) was initiated.

## Claims

1. Use of a permeation enhancing amount of methylsulfonylmethane for the preparation of a formulation containing ciprofloxacin, and a pharmaceutically acceptable ophthalmic carrier, for delivering ciprofloxacin to the eye of a patient, wherein the permeation enhancing amount of methylsulfonylmethane is in the range of 1.0 wt.% to 33 wt.% of the formulation.

2. The use of claim 1, wherein the permeation enhancing amount of methylsulfonylmethane is an amount effective to enhance penetration of formulation components through the membranes and tissues of the eye.

3. The use of claim 1, wherein the permeation enhancing amount of methylsulfonylmethane is an amount effective to transport formulation components to the anterior and posterior of the eye.

4. The use of claim 1, wherein the permeation enhancing amount of methylsulfonylmethane is in the range of 1.5 wt.% to 8.0 wt.% of the formulation

5. The use of claim 1, wherein the pharmaceutically acceptable ophthalmic carrier is at least partially aqueous.

6. The use of claim 1, wherein the formulation comprises a solution, suspension, hydrogel, dispersion, or ointment.

7. A formulation for delivering an ophthalmologically active agent to the eye of a patient, the formulation **characterized in that** the formulation contains, in addition to an effective amount of an ophthalmologically active agent, a pharmaceutically acceptable ophthalmic carrier, a permeation enhancing amount of methylsulfonylmethane in the range of 1.0 wt.% to 33 wt.% of the formulation, wherein the ophthalmologically active agent is ciprofloxacin.

8. The formulation of claim 7, wherein the permeation enhancing amount of methylsulfonylmethane is an amount effective to enhance penetration of formulation components through the membranes and tissues of the eye.

9. The formulation of claim 7, wherein the permeation enhancing amount of methylsulfonylmethane is an amount effective to transport formulation components to the anterior and posterior of the eye.

10. The formulation of claim 7, wherein the permeation enhancing amount of methylsulfonyfmethane is in the range of 1.5 wt.% to 8.0 wt.% of the formulation

11. The formulation of claim 7, wherein the pharmaceutically acceptable ophthalmic carrier is at least partially aqueous.

12. The formulation of claim 7, wherein the formulation comprises a solution, suspension, hydrogel, dispersion, or ointment.

## Patentansprüche

1. Verwendung einer permeationsverbessernden Menge von Methylsulfonylmethan für die Herstellung einer Formulierung, die Ciprofloxacin und einen pharmazeutisch verträglichen ophthalmischen Träger enthält, zum Zuführen von Ciprofloxacin zu dem Auge eines Patienten, wobei die permeationsverbessernde Menge von Methylsulfonylmethan in dem Bereich von 1,0 bis 33 Gew.-% der Formulierung liegt.

2. Verwendung nach Anspruch 1, wobei die permeationsverbessernde Menge von Methylsulfonylmethan eine Menge ist, die eine Verbesserung der Penetration von Formulierungsbestandteilen durch die Membranen und Gewebe des Auges bewirkt.

3. Verwendung nach Anspruch 1, wobei die permeationsverbessernde Menge von Methylsulfonylmethan eine Menge ist, die den Transport von Formulierungsbestandteilen zu dem vorderen und hinteren Augenabschnitt bewirkt.

4. Verwendung nach Anspruch 1, wobei die permeationsverbessernde Menge von Methylsulfonylmethan in dem Bereich von 1,5 bis 8,0 Gew.-% der Formulierung liegt.

5. Verwendung nach Anspruch 1, wobei der pharmazeutisch verträgliche ophthalmische Träger wenigstens teilweise Wasser enthält.

6. Verwendung nach Anspruch 1, wobei die Formulierung eine Lösung, eine Suspension, ein Hydrogel, eine Dispersion oder eine Salbe umfasst.

7. Formulierung zum Zuführen eines ophthalmologisch aktiven Wirkstoffs zu dem Auge eines Patienten, wobei die Formulierung **dadurch gekennzeichnet ist, dass** sie zusätzlich zu der wirksamen Menge eines ophthalmologisch aktiven Wirkstoffs einen pharmazeutisch verträglichen ophthalmischen Träger und eine permeationsverbessernde Menge von Methylsulfonylmethan in dem Bereich von 1,0 bis 33 Gew.-% der Formulierung enthält, wobei der ophthalmologisch aktive Wirkstoff Ciprofloxacin ist.

8. Formulierung nach Anspruch 7, wobei die permeationsverbessernde Menge von Methylsulfonylmethan eine Menge ist, die eine Verbesserung der Penetration von Formulierungsbestandteilen durch die Membranen und Gewebe des Auges bewirkt.

9. Formulierung nach Anspruch 7, wobei die permeationsverbessernde Menge von Methylsulfonylmethan eine Menge ist, die den Transport von Formulierungsbestandteilen zu dem vorderen und hinteren Augenabschnitt bewirkt.

10. Formulierung nach Anspruch 7, wobei die permeationsverbessernde Menge von Methylsulfonylmethan in dem Bereich von 1,5 bis 8,0 Gew.-% der Formulierung liegt.

11. Formulierung nach Anspruch 7, wobei der pharmazeutisch verträgliche ophthalmische Träger wenigstens teilweise Wasser enthält.

12. Formulierung nach Anspruch 7, wobei die Formulierung eine Lösung, eine Suspension, ein Hydrogel, eine Dispersion oder eine Salbe umfasst.

## Revendications

1. Utilisation d'une quantité de méthylsulfonylméthane améliorant la perméation pour la préparation d'une formulation contenant de la ciprofloxacine et un support ophtalmique pharmaceutiquement acceptable, pour administration de ciprofloxacine à l'oeil d'un patient, la quantité de méthylsulfonylméthane améliorant la perméation étant dans la plage de 1,0 % en poids à 33 % en poids de la formulation.

2. Utilisation selon la revendication 1, dans laquelle la quantité de méthylsulfonylméthane améliorant la perméation est une quantité efficace pour améliorer la pénétration des composants de la formulation à travers les membranes et tissus de l'oeil.

3. Utilisation selon la revendication 1, dans laquelle la quantité de méthylsulfonylméthane améliorant la perméation est une quantité efficace pour transporter les composants de la formulation vers les parties antérieures et postérieures de l'oeil.

4. Utilisation selon la revendication 1, dans laquelle la quantité améliorant la perméation de méthylsulfonylméthane est dans la plage de 1,5 % en poids à 8,0 % en poids de la formulation.

5. Utilisation selon la revendication 1, dans laquelle le support ophtalmique pharmaceutiquement acceptable est au moins partiellement aqueux.

6. Utilisation selon la revendication 1, dans laquelle la formulation comprend une solution, une suspension, un hydrogel, une dispersion ou une pommade.

7. Formulation pour administrer un agent actif ophtalmologiquement à l'oeil d'un patient, la formulation étant **caractérisée en ce qu'**elle contient, outre une quantité efficace d'un agent actif ophtalmologiquement, un support ophtalmique pharmaceutiquement acceptable, une quantité de méthylsulfonylméthane améliorant la perméation dans la plage de 1,0 % en poids à 33 % en poids de la formulation, l'agent actif ophtalmologiquement étant de la ciprofloxacine.

8. Formulation selon la revendication 7, dans laquelle la quantité de méthylsulfonylméthane améliorant la perméation est une quantité efficace pour améliorer la pénétration des composants de la formulation à travers les membranes et tissus de l'oeil.

9. Formulation selon la revendication 7, dans laquelle la quantité de méthylsulfonylméthane améliorant la perméation est une quantité efficace pour transporter les composants de la formulation vers les parties antérieures et postérieures de l'oeil.

10. Formulation selon la revendication 7, dans laquelle la quantité améliorant la perméation de méthylsulfonylméthane est dans la plage de 1,5 % en poids à 8,0 % en poids de la formulation.

11. Formulation selon la revendication 7, dans laquelle le support ophtalmique pharmaceutiquement acceptable est au moins partiellement aqueux.

12. Formulation selon la revendication 7, dans laquelle la formulation comprend une solution, une suspension, un hydrogel, une dispersion ou une pommade.
